# EUROPEAN PATENT SPECIFICATION

(11) **EP 1 862 556 B1**
(45) Date of publication and mention of the grant of the patent: **20.10.2010**
(21) Application number: 06090096.6
(22) Date of filing: 02.06.2006
(51) Int. Cl.: C12Q 1/68

(54) **Method for identifying genetic markers for secondary tumors and means for the identification, labelling and targeting of secondary tumors**
Verfahren zu der Identifizierung von genetischen Markern für Sekundärtumore und Mittel zur Identifizierung, Markierung und zur gezielten Behandlung von sekundären Tumoren
Méthode pour identifier des marqueurs génétiques de tumeurs secondaires et moyens pour l'identification, le marquage et le ciblage de tumeurs secondaires

(43) Date of publication of application: 05.12.2007
(73) Proprietor: Fraunhofer-Gesellschaft zur Förderung der angewandten Forschung e.V., 80686 München (DE)
(72) Inventor: Borlak, Jürgen, Prof. Dr., 31275 Lehrte OT Immensen (DE); Lehner, Frank, Dr., 30916 Isernhagen (DE); Klempnauer, Jürgen, Prof. Dr., 31515 Steinhude (DE)
(74) Representative: Baumbach, Friedrich

(56) References cited:
- EP-A2- 0 284 362
- EP-A2- 1 271 153
- WO-A2-2004/063355
- DATABASE BIOSIS [Online] BIOSCIENCES INFORMATION SERVICE, PHILADELPHIA, PA, US; 1996, SAMADANI UZMA ET AL: "The transcriptional activator hepatocyte nuclear factor 6 regulates liver gene expression" Database accession no. PREV199699259808 & MOLECULAR AND CELLULAR BIOLOGY, vol. 16, no. 11, 1996, pages 6273-6284, ISSN: 0270-7306

## Description

The invention relates to a method for identifying therapeutical targets in secondary tumors, such as in metastases in the liver made up of or derived from non-hepatic tumor cells, the use of novel therapeutical targets identified by the method for screening and determining means and/or drugs, and means and drugs for identifying, labeling and treating secondary metastases in the liver made up of or derived from tumor cells of the colon. Areas of application are the life sciences: biology, biochemistry, biotechnology, medicine and medical technology.

In comparison with primary tumors (e.g. hepatocellular carcinoma, cholangiocellular carcinoma), where the organ by itself is the starting point of the malignant degeneration, metastases inherit a different emergence. For example, in the processes of malignant diseases liver metastases are observed in up to 50% of the cases. Thus, the liver represents the most frequent localization of organ metastases, such as from malignant tumors of the lung, breast, skin, colon, kidney or prostate.

Particularly, gastrointestinal malignomes show hepatic metastasis due to their venous drainage over the portal vene. After diagnosis, the mean life expectancy amounts to usually less than 8 months, in the spontaneous process a long-term surviving (> 5 years) is only rarely observed. The only chance for healing exists, so far, in the resection of the metastases in the healthy tissue. However, only a small part of all patients concerned can profit from a surgical treatment, since either a generalized metastasis is already present, or - in the isolated hepatic infestation - it is often technically difficult to resect the tumor source.
Therefore, in practice, metastases of colorectal carcinomes are almost exclusively considered for a curative resection, since the liver represents the first filter organ and is often the only localization of metastases due to functioning as a portal-venous drainage. In addition, only about 20-25% of all colorectal liver metsatsases can be supplied to a curative (so-called R0) resection. Such curative resections of liver metastase from other primary tumors is, however, more rarely possible. Even after a R0-resection of liver metastases of a colonic carcinome, 60-70% of the patients develop a tumor relapse.

This means, that only for a minority of the patients a surgical therapy of liver metastases can be taken into consideration as a curative therapy.

Colon cancer is the second leading cause of cancer death with over 140.000 cases diagnosed annually for the United States alone.¹ A frequent complication of this malignancy is the development of colorectal liver metastases which results in impaired liver function and eventually hepatic failure.² Despite improved methods for detection and treatment the patient's overall 5 year survival remains poor, ranging from 24 to 44%.³ Research is needed to improve an understanding of the molecular causes of colorectal liver metastases and to identify novel opportunities for the treatment of this secondary liver malignancy.

In the year 2001 the decoding of the human genome was published. The function of individual genes is well-known for approx. 21000 genes. It could be shown that approx. 2500 transcription factors steer the transcriptional regularization of the human genome. Further, it could be shown that in many cases genetic patterns or changes are responsible for the emergence of malignant processes. Thereby, already in some cases, the possibility of measuring the individual risk for evolving a malignant disease exists by suitable genetic analyses.

The document WO 2004/063355 A2 provides nucleotide sequences of genes that are up- and down-regulated in metastatic breast or metastatic lung cancer cells (page 2, lines 24-25). As disclosed on page 27, lines 8-13 of WO 2004/063355 A2, a metastatic breast cancer or metastatic lung cancer screen may be performed for identifying metastatic breast cancer or metastatic lung cancer-associated sequences, including comparing genes identified in different tissues, e. g., normal and cancerous tissues, or tumor tissue samples from patients who have metastatic disease vs. non metastatic tissue, or tumor tissue samples from patients who have been diagnosed cancer but have survived vs. metastatic tissue. Other suitable tissue comparisons include comparing metastatic breast cancer or metastatic lung cancer samples with metastatic cancer samples from other cancers, such asgastrointestinal cancers, prostate, ovarian, etc. (page 27, lines 13-15 of WO 2004/063355 A2).

However, the molecular causes leading to secondary liver malignancies are unknown so far.

The aim of the present invention is therefore to make available an easy and efficient method for identifying secondary metastases in the liver made up of or derived from tumor cells of the colon. To this end, the implementation of the actions as described in the claims provides appropriate means to fulfill these demands in a satisfying manner.

Thus, the invention in its different aspects and embodiments is implemented according to the claims.

The inventive method for identifying therapeutical targets in secondary tumors is based on the ability of tumour cells to adapt to the local environment necessitates plasticity. This, in turn, requires a complex and timely interplay of transcription factors, which are master regulatory proteins and interact with many different molecules including coactivators, repressors, enzymes, DNA and RNA to control gene expression. These interactions will inevitably repress or activate gene expression and thus determine cellular phenotype. For example, numerous studies have established the pivotal role of liver-enriched transcription factors in organ development and liver function and there is conclusive evidence for transcription factors to act in concert to enable cellular differentiation and regulation of metabolic functions.⁴⁻⁷ Some of these transcription factors are tissue specific; but others are required for gene expression in a variety of tissues. In general, liver enriched transcription factors are classified by their ability to recognize specific DNA binding motifs and are divided into major families, e.g. hepatocyte nuclear factors 1, 3, 4 and 6 and their isoforms as well as the CAAT/enhancer binding proteins including the many subfamily members, as reviewed.^{8,9}

In principle, expression of transcription factors is studied according to the inventive method, in particular of transcription factors being enriched in the healthy tissue of the organ wherein the secondary tumor is formed and/or found, e.g. expression of liver enriched transcription factors is studied, such as by reverse transcription polymerase chain reaction, by gene chip analysis, by Western blotting technique, by studying the DNA binding of liver enriched transcription factors by electromobility shift assay (EMSA) or by genomic sequencing of therapeutical targets, such as of HNF6.

In the following
- a RNA sample isolated from the tissue of a primary tumor is also designated as "RNA (1)"
- a RNA sample isolated from the tissue of an organ, wherein the secondary tumor is formed is also termed as "RNA (2)"
- a RNA sample from the healthy tissue of an organ, wherein the secondary tumor is formed, is also designated as "RNA (3)".

Tissues are preferably resected or isolated from the tumors and organs by standard procedures or other known methods of resection or isolation. According to the invention the term "tissue" comprises cellular material of tumors and organs in all different forms, e.g. tissue dices, cells, cell compartiments, or homogenisate of tissues, cells or cellular compartments. For example, resected tissues can be lysed as a whole or be seperated in cells, the latter providing the beneficial possibility of amplifyng the cellular material of the tumor in cell culture before cell lysis. Hence, in principle only one cell of the primary tumor tissue and one cell of the secondary tumor tissue is sufficient for receiving approriate amounts of RNA to be isolated. Tissues used are preferably resected or isolated from solid organs, e.g. liver, brain or bone and/or from solid tumors, e.g of the lung, breast, skin, colon, kidney or prostate, liver, brain or bone. In particular, the term "solid organs" concerns all larger voluminous biological structures made up of mainly homogenous tissue and the term "solid tumors" concerns all primary and/or secondary tumors being formed in solid organs.

The RNA to be isolated can be purified from the tissues, in particular mammalian tissues, according to standard procedures or other known methods, such as by using RNA isolation kits. According to the invention, standard RNA isolation procedures have the advantegous characteristic, that they result in a solution including the transcriptome of the respective tissue, i.e the set of all mRNA molecules (or *transcripts*) in one or a population of the tissue cells for the given set of environmental circumstances, thus allowing the detection of all transcripts of transcription factors being present in the tissue at a time.

The transcription factors to be screened according to the invention are proteins that bind DNA, in particular at a specific promotor or enhancer region or site, where they regulate transcription. The transcription factors to be screened are, in particular, general transcription factors, upstream transcription factors or inducible transcription factors. Transcription factors appropriate for fulfilling the invention typically comprise at least one motif found in transcription factors such as a helix-turn-helix, a zinc finger, a leucine zipper, a basic-helix-loop-helix, a G-quadruplex motif or an intrinsically disordered region essential for transcriptional regulation [Minezaki Y. et al. J Mol Biol 2006 Apr 25 [Epub ahead of print]. Typically, the transcription factors to be screened for fulfilling the invention are selected from a database, e.g. the DBD database or from other publications or are already inherently included in the means for determining the expression levels, such as in DNA microarrays for a transcriptomic approach. The transcription factors being enriched in the healthy tissue are either determined, such as by comparing RNA (3) with RNA isolated from a different organ species, preferably by using array techniques, e.g. DNA microarrays, or are chosen from databases or from data published elsewhere.

For each of the isolated RNA (1) - (3), a gene expression profile of at least two genes coding for different transcription factors, in particular coding for the transcription factors being enriched in the healthy tissue, is determined by screening the presence of mRNA coding for the transcription factors to be screened and by determining the levels of expression of thereof. The term "level of expression" according to the invention in particular concerns the amount of mRNA transcripts being present in a transcriptome.

The expression profiles to be determined according to the invention are performed by standard procedures of expression profiling or other known methods for parallely determining the presence of several different mRNA molecules in an isolated RNA sample, e.g. by PCR methods for amplifying and/or synthesizing oligonucleotides and by determination (qualitatively and/or quantitatively) of the such produced oligonucleotides using (gel) electrophoretic separation or array techniques and subsequently registering, imaging, estimating, and/or calculating the present amounts of transcription factors in the samples tested (levels of expression). For imaging purposes all possible and standard imagers and scanners for vizualizing separated or spatially enriched oligonucleotides, e.g. a Lumi-imager or a microarray scanner, are suitable, thus allowing an easy adaption of the invention to different laboratory equipments.

The gene expression profiles determined according to the invention are mutually compared, by standard procedures or other known methods for comparing the results of gene expression profilings, in a manner that the profile of one tissue tested is compared with the profile of the two further tissue species tested. The gene expression profiles are thus pairwisely compared and the results are joint and/or correlated, so that the overall expression status of the transcription factors can be determined as being
(a) upregulated or enriched in the three different RNA samples RNA (1), RNA (2), and RNA (3) (termed as "RNA (1) - (3)") or
(b) downregulated or non detectible in RNA (1) - (3).

For example, a transcription factor is downregulated in RNA (1), upregulated in RNA (2), and upregulated in RNA (3) if
- no signal(s) or low signal intensity for the transcription factor (e.g. a missing specific band in the agarose gel or a missing specific array signal) is detectable in the gene expression profile for RNA (1),
- signal(s) for the transcription factor is detectable (e.g. by a specific band in the agarose gel or a specific array signal) in the gene expression profile of RNA (2) or signal intensity is significantly higher (enriched) in the gene expression profile of RNA (2) than in the gene expression profile of RNA (1).
- signal(s) for the transcription factor is detectable (e.g. by a specific band in the agarose gel or a specific array signal) in the gene expression profile of RNA (3) or signal intensity is significantly higher (enriched) in the gene expression profile of RNA (3) than in the gene expression profile of RNA (1).

In the same manner, any other values or levels, e.g. the level of expression, deduced from the signal(s) or signal intensities is approriate for classifying the transcription factor as being upregulated or downregulated. In this context preferably a (molecular) standard, e.g. a transcript, such as of mitochondrial ATPase, being detectable in each of the RNA (1) - (3), is parallely used or determined for standardizing the results received.

The afore mentioned principle of pairwisely evaluating signals or values is also analogously perfomed for the correlation or comparison of proteins and fragments of thereof, as being described beneath.

A marker is identified according to the invention as a transcription factor being downregulated or mainly non-detectible in the gene expression profile of the isolated RNA (1), upregulated or enriched in the gene expression profile of the isolated RNA (2), and upregulated or enriched in the gene expression profile of the isolated RNA (3).

Preferably RNA (1) is isolated from the tissue, in particular from human or rodent tissue; of a malignant tumor of the lung, breast, skin, colon, kidney or prostate, RNA (2) is isolated from the tissue, in particular from human or rodent tissue, of a metastatic tumor spread from the malignant tumor into the adrenal, liver, brain or bone and RNA (3) is isolated from the healthy tissue, in particular from human or rodent tissue, of the adrenal, liver, brain or bone, thus allowing to implement the invention specifically to different forms of secondary tumors. For example, RNA (1) is isolated from a primary tumor of the skin of a human patient, RNA (2) is isolated from a secondary tumor being spread from the skin into the liver of this human patient, and RNA (3) is isolated from healthy liver tissue of a different human being or from healthy liver tissue of the same patient. The latter provides the favourable characteristic that therapeutical targets can be individually identified for a patient, allowing a tailored diagnostic and treatment of the patient suffering from the metastase(s) in his/her liver.

In particular, for identifying therapeutical targets in metastases in the liver made up of or derived from non-hepatic tumor cells, RNA (1) is isolated from the tissue of a primary colonic tumor of a human patient, RNA (2) is isolated from the tissue of the colorectal liver metastasis of a human patient, RNA (3) is isolated from the healthy tissue of a human liver and the gene expression profile of at least two genes coding for transcription factors being enriched in the healthy liver tissue is determined for each of the isolated RNA (1) - (3).

Another preferable aspect concerns the inventive method, wherein the gene expression profile of at least two genes selected from the group of genes in Table 2 is determined, allowing a fast and efficient identification procedure and further reduces the screening expense.

In yet another aspect, the expression profiling comprises the syntheses of three cDNA libraries, in the following designated as cDNA libraries (4), (5), (6), each of which being derived of a different isolated RNA (1) - (3), by a reverse transcription polymerase chain reaction (RT-PCR) enabeling an easy first strand reaction.

Yet another aspect of the invention concerns the gene expression profiling which further comprises the steps of amplifying cDNA sequences of the transcription factors to be screened by a PCR, in particular by using a thermocycler, wherein each of the cDNA libraries (4) - (6) is used in a mixture with synthetic primers being, at least in parts, complementary with the transcription factors to be screened, the primers being preferably selected from the group of the primers in Table 2, and separating the amplified cDNA sequences by gel electrophoresis of the PCR reaction mixtures and vizualizing the separated PCR products. This aspect allows a straightforward approach for fulfilling the invention in a specific sensitive manner.

Preferably, for vizualizing the separated PCR products, such as by standard procedures, labeled synthetic primers are used in the PCR, or a substance, in particular a dye such as ethidium bromide may be, intercalating in double stranded oligonucleotides, is used for labelling the PCR products, thus enabeling an easy identification of oligonucleotides.

A further aspect of the inventive method relates to the gene expression profiling being implemented by the steps of synthesizing three cRNA libraries (7), (8), (9), each of which being derived of a different cDNA library (4) - (6) by second strand cDNA synthesis and by *in vitro* transcription of the double stranded cDNA, producing three RNA fragment libraries (10), (11), (12), each of which being derived of a different cRNA library (7) - (9) by hydrolytic cleavage into RNA fragments, e.g. by metal-induced hydrolysis into RNA fragments of the length of 35-200 bases, performing hybridization assays by incubating three, at least in parts, identical oligonucleotide arrays (13), (14), (15), including spatially addressed solid phase bound oligonucleotide sequences coding for the at least two transcription factors to be screened or for parts of thereof, each of which with a solution including a different cRNA fragment library (10) - (12), and scanning the hybridization patterns of the oligonucleotide arrays (13) - (15). This aspect of the invention allows a fast and efficient detection and/or evaluation of a large number of different transcription factor transcripts, in particular if labelled ribonucleotides, such as biotin labelled ribonucleotides may be, are used for the *in vitro* transcription, and/or oligonucleotide microarrays , e.g. DNA microarrays, are used for performing the hybridization assays.

Another aspect of the invention concerns the inventive method, which further comprises the steps of isolating a total protein extract from the tissue of the secondary tumor, in the following also termed as protein extract (16), and isolating a total protein extract (protein extract (17)) from the healthy tissue of an organ, wherein the secondary tumor is formed. This aspect has the advantegous characterisitc that it enables the determining of the effectively expressed proteins in the proteome(s) of tissue cells, since translation of the mRNA can be blocked by different factors in the cell.

Yet a further aspect relates to the inventive method further comprising, in particular by the use of an ultracentrifuge according to standard or other known methods for isolating biological fractions in a centrifugal field, the steps of isolating nuclei from the tissue of the primary tumor, also subsequently termed as nuclei (18), isolating nuclei from the tissue of the secondary tumor (nuclei (19)) and isolating nuclei (nuclei (20)) from the healthy tissue of the organ, wherein the secondary tumor is formed, and isolating protein extracts (21), (22), (23) and/or isolating DNA extracts (24), (25), (26) from the isolated nuclei (18) - (20), thus allowing a precise determination of proteins, in particular of transcription factors, being present and/or active in the nucleus and/or of DNA usable for binding studies.

In another aspect the inventive method further comprises a Western immunoblotting of the protein extracts (16) and (17) and/or of the protein extracts (21) - (23), thus enabeling a separation, vizualization and/or analysis of the proteins, in particular if monoclonal and/or polyclonal antibodies being directed against at least one transcription factor to be screened, in particular being directed against a therapeutical target determined according to the inventive method and/or against the native ligands of thereof, such as antibodies being directed against HNF6, are used. In particular, for the protein extracts (21)-(23), the immunoblotting patterns are pairwisely compared.
A further aspect relates to the inventive method, wherein a closer characterization of the identifid target(s) is accomplished. Non-posttranslationally modified target and the corresponding posttranslationally modified target(s), e.g. acetylated and non-acetylated HNF6, are separated and/or isolated from the protein extracts (16) and (17), such as by chromatographic (e.g. FPLC), electrophoretic (e.g. gel electrophoresis) and/or array techniques (e.g. protein and/or peptide arrays) and/or are characterized, such as by an immunoassay, e.g. ELISA, sequencing, e.g. protein/peptide sequencing, and/or mass spectometry, e.g. MALDI or ESI, for determining the presence and/or the amount(s) of thereof, and if the posttranslationally modified target(s) is detectable and/or significantly higher (elevated) in the protein extract (17) compared with the protein extract (18), this target species, e.g. acetylated HNF6, is determined as the precised target to be upregulated in the secondary tumor. Examplarily according to the invention, acetylated HNF6 is determined as the target species to be upregulated, e.g. by therapeutical means, in colorectal liver metastases.

In another preferred embodiment, a post translationally modified gene product and/or its post translationally modified variant and/or part thereof and/or a derived sequence of thereof is employed for the inventive use, such as acetylated HNF6 gene product and/or an acetylated mutant and/or variation and/or part thereof and/or a derived sequence of thereof is used.

Another aspect of the invention relates to a procedure for identifying, liver metastases, in particular metastases in the liver made up of or derived from non-hepatic tumor cells, such as colorectal liver metastases wherein a biological system is contacted with a soluble substance having affinity with HNF6, and/or its variants and/or parts thereof wherein the soluble substance is linked with a marker.

As the, at least partially, soluble substance in particular oligonucleotides, proteins, peptides or structures derived of thereof are suitable. These have the advantage that they can recognize specifically two or three-dimensional target structures on the molecular level. Beyond that, they provide the favourable characteristic that the recognition usually takes place in aqueous physiologically buffered solutions and leads to a specific association/binding with the targeted structure.
Thereby, monoclonal and/or polyclonal antibodies and/or antibody fragments are particularly suitable, since they are formed as stable highly specific structures, which are, in principle, producible against all possible molecular target structures. In a favourable embodiment of the procedure human and/or bispecific antibodies or human and/or bispecific antibody fragments are used.
In particular, monoclonal antibodies and/or antibody fragments are thereby suitable.
For implementing the invention it is preferred, if the marker according to invention is selected as an element, an isotope, a molecule and/or an ion or is composed of thereof, such as a dye, contrast means, chemotherapeutic agent, radionuclide, toxin, lipid, carbohydrate, biotin, peptid, protein, microparticle, vesicle, polymer, hydrogel, cellular organelle, virus and/or whole cell, in particular if the marker is formed as dye labeled and/or enzyme-labeled secondary antibodies and/or as protein A and/or as protein G or structures derived of thereof.
The linkage between the marker and the substance is favourably chemically, electrostatically and/or over via hydrophobic interactions, such as there is sufficient connection stability for the use of the marked substance for identifying, labelling and treating of metastatic cells, preferably if the linkage is covalent.
For the increase of the sensitivity of the procedure according to invention also the simultaneous use of several substances is in particular suitable.
In particular, for a specific recognition/identification substances are used, which bind with an affinity above the association constant Ka = 1000 M-1 to the target structure.

For implementing the procedure according to the invention it is favourable to use one of the following methods - PCR, in vitro translation, RT-PCR, gel electrophoresis, Western Blot, Northern Blot, Southern Blot, ELISA, FACS measurement, chromatographic isolation, UV microscopy, immunohistochemistry, screening of solid phase bound molecules or tissues and/or biosensory investigation - whereby by amplification, isolation, immobilization and/or detection and/or by combinations of thereof a particularly simple conversion of the procedure according to invention is made possible for the examined sample, in particular if furthermore a statistic analysis is accomplished.

The procedure according to invention is preferably implemented by using molecules, cells and/or tissue, in particular being immobilized or synthesized on a planar surface, e.g. spatially addressed. on a nitrocellulose or PVDF membrane, or is linked to the cavity of a microtiter/ELISA plate or on the bottom of a cell culture container, in particular on a glass or plastic chip (biochip).
Favourably, as solid phase bound molecules, substances are used, having affinity for at least one and in particular HNF6 and/or its variants and/or parts of thereof.
The indentification of target structures, e.g. of transcription factors of an immobilized section of tissue or of cells of a cell culture, can take place thereby with arbitrarily marked molecules, which are brought on the surface in solution, e.g. (fluorescence marked/labeled) antibodies.
If a RT-PCR is accomplished, then favourably appropriate oligonucleotide probes and/or primers are used. For implementing the procedure according to invention immobilized molecule libraries, e.g. DNA or antibody libraries are used, which associate with the target structure(s) in solution, e.g. with a cDNA library, a PCR product library or with cells of a secondary tumor.
Substances bound to the molecule libraries are particularly identified by the use of appropriately marked probes, e.g. dye labeled oligonucleotides. If unabeled primary antibodies are used, preferably labeled secondary antibodies are used for detection. Furthermore, the use of other proteins, e.g. enzymes, or streptavidin or parts of thereof is suitable. For the diagnosis of a secondary tumor, in particular by *in vitro* and/or *in vivo* diagnostics, with the help of the procedure according to invention preferably optically (or radiographically) sensitive equipment, is used, e.g. an UV microscope, scanner or ELISA reader, photometer, or szintigrafic equipment, e.g. X-ray gadget are appropriate.

A further aspect concerns the inventive procedure, wherein the biological or biotechnological system used is an organism, a tissue, a cell, a part of a cell, a DNA, a RNA, a cDNA, a mRNA, a cRNA, a protein and/or a peptide and/or a derived structure and/or contains the same, such as cells of a liver metastasis and/or an oligonucleotide library.

Yet another aspect of the inventive procedure is related to a substance having specific affinity with the post translationally acetylated HNF6 gene product.

Also, the invention concerns means for the identification, of secondary tumors, having affinity with HNF6, its variants, such as antibodies being directed against HNF6.

Other features and advantages will become apparent from the following detailed description.

In the following, the concept and proof of the invention is exemplarily shown for metastases in the liver made up of or derived from non-hepatic tumor cell, but the invention is, in analogy, appropriate to all different forms of secondary tumor malignacies, as described herein.

In comparison with primary liver tumors (e.g. hepatocellular carcinoma, cholangiocellular carcinoma), where the liver by itself is the starting point of the malignant degeneration, liver metastases inherit a different emergence. In the processes of malignant diseases liver metastases are observed in up to 50% of the cases. Thus, the liver represents the most frequent localization of organmetastases, such as from malignant tumors of the lung, breast, skin, colon, kidney or prostate.

As of today, the expression of liver enriched transcription factors in human colorectal liver metastases and in primary tumours of the colon is unknown. Conceivably, expression of transcription factor during disease changes with important implications for the transcriptional network of genes targeted by these factors thereby impacting cellular phenotype.

In the work leading to the invention the regulation of major hepatic nuclear factors in primary human colonic cancer and colorectal liver metastases was investigated.

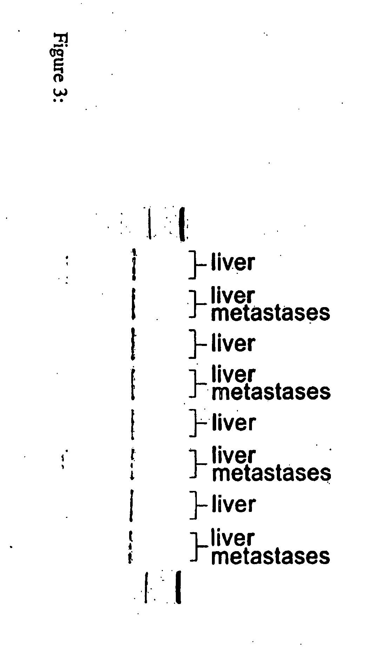

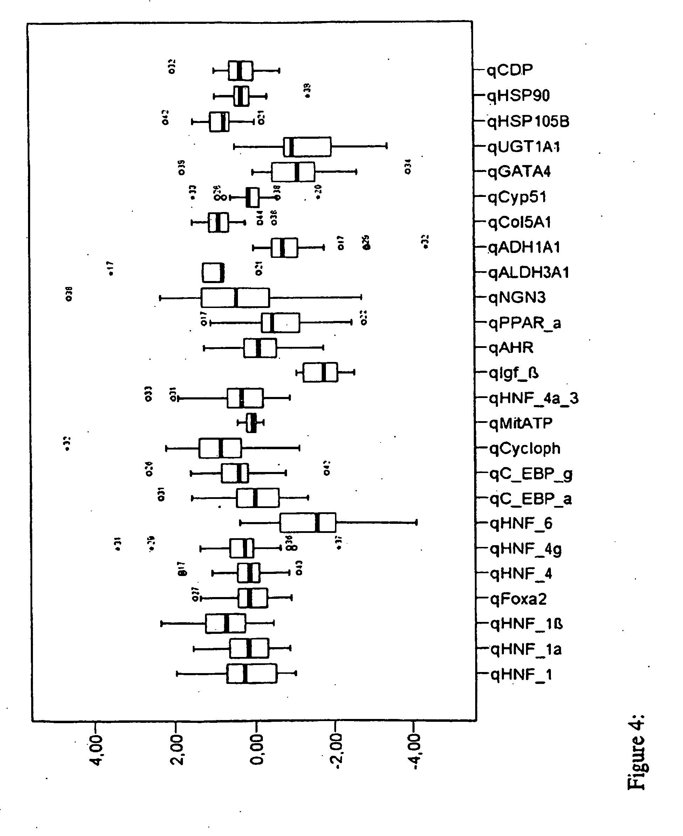

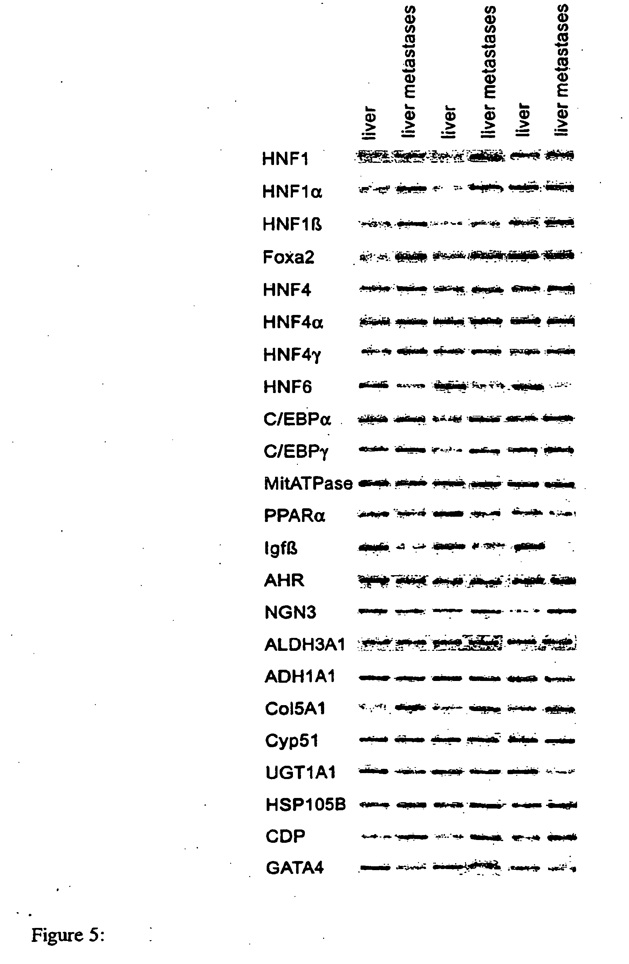

As an example for fulfilling the invention, the gene expression of liver enriched transcription factors in healthy liver and tumour tissue was studied. Gene expression of transcription factors was computed relative to the housekeeping gene mitochondrial ATPase, which were found to be stable expressed (Fig. 3). Expression of liver enriched transcription factors did not differ statistically (Fig. 4) except for HNF6, HNF1β and C/EBPγ. Furthermore, abundance of transcript expression of most transcription factors was less when compared with that of mitochondrial ATPase. Expression of transcription factors varied amongst individual patients. Fig. 5 depicts a representative ethidium bromide stained RT-PCR gel for some patients. Expression of hepatic nuclear factors differed, when healthy liver and colorectal liver metastases was compared, but did not reach statistical significance, as observed with Foxa2, HNF4, HNF4α, HNF4γ, CEBPα, CEBPβ, CDP and GATA4.

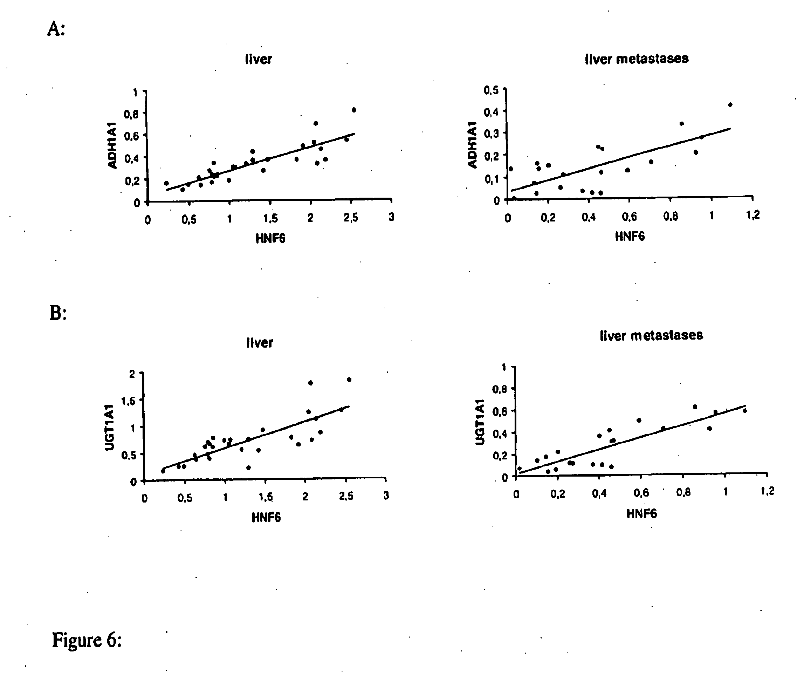

Additionally the expression of ALDH3A1, ADH1A1, Co15A1, Cyp51, HSP105, UGT1A1 was studied in healthy liver and tumourous tissue (Fig. 4). These genes are bona fide targets of HNF6 ¹¹. Based on computational analysis (Spearman's correlation coefficient) expression of HNF6 and ADH1A1 and/or UGT1A1 was found to be significantly regulated in the patient cohort. Fig. 6 depicts scatter blots for n=29 patients. Note, the diagrams represent a relationship for either healthy or tumourous tissue. In addition, HNF6 serves as a coactivator protein to enhance Foxa2 (=HNF3β) transcription. At the gene expression level no significant correlation between HNF6 and Foxa2 expression was obtained (scatter blots not shown).

The expression of neurogenin 3 (NGN3) was studied, which is a bona fide target of HNF6.^{12,13} Specifically, NGN3 is a transcription factor required for the specification of the endocrine lineage in uncommitted and multipotent intestinal progenitor cells. Mice homozygous for a null mutation in ngn3 fail to generate any intestinal endocrine cells as well as endocrine progenitor cells. There was considerable variation in the expression of NGN3 in healthy liver and colorectal liver metastases with the mean expression of NGN3 being higher in tumour tissue. This suggests additional regulation of NGN3 in colorectal liver metastases which appears to be independent of HNF6.

The gene expression of HNF6 and of heat shock proteins HSP105B and HSP90 significantly correlated in colorectal liver metastases. These genes are bona fide targets for HNF6 and act as chaperone in facilitating protein folding. Indeed, induction of proteosomal degradation of HSP90 super chaperone complexes is clinically perused to abrogate oncogenic protein expression.¹⁴ There was tight regulation between HNF6 and of the gene coding for collagen5A1 in healthy liver tissue, but less so in liver tumours. Finally, gene expression of CCAAT enhancer displacement protein (=CDP) and of GATA4 in healthy liver tissue and colorectal liver metastases was studied. Notably, COP may act as a competitive repressor for CCAAT protein mediated transactivation of targeted genes9 and competes for binding sites of CEBP's in order to repress histone deacetylase activity. CDP gene expression was found to be statistically significant induced in colorectal liver metastases (see box blot Fig. 4).

Furthermore, the GATA family of Zn-finger transcription factors participates in gastrointestinal development. Recent evidence suggests epigenetic silencing of GATA4 and GATA5 in colorectal and gastric cancer through promoter hypermethylation of CpG islands_{.}¹⁵
GATA4 gene expression was found to be significantly reduced in colorectal liver metastases and it was not possible to amplify GATA5 transcripts in healthy or tumourous tissue.
Further, gene expression of insulin-like growth factor (IGF1) was investigated in the patient cohort and IGF1 mRNA levels were found to be significantly reduced in colorectal liver metastases, when compared with healthy liver tissue of the same patient.

### Gene expression profiling in primary tumours of the colon

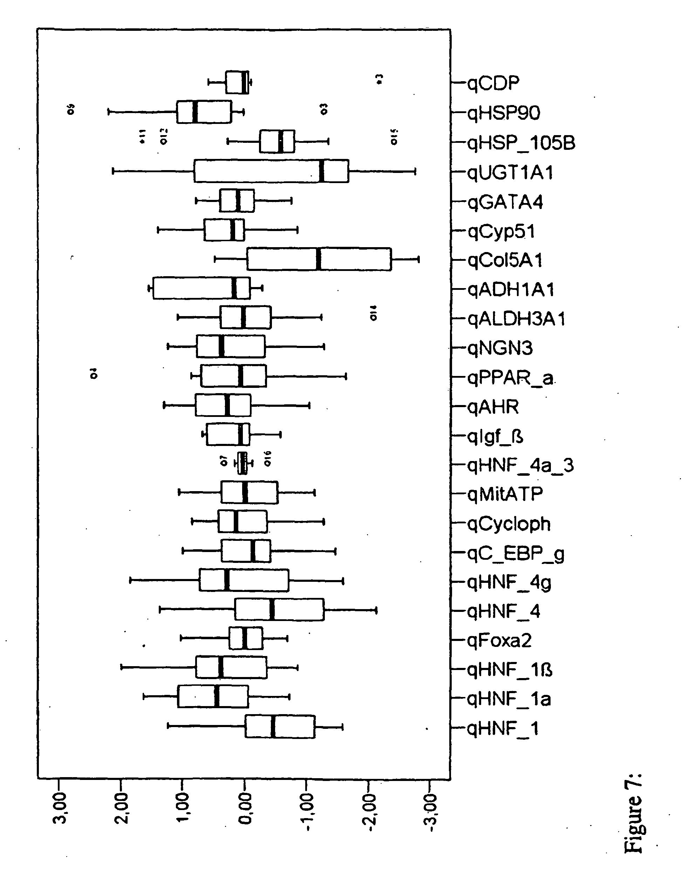

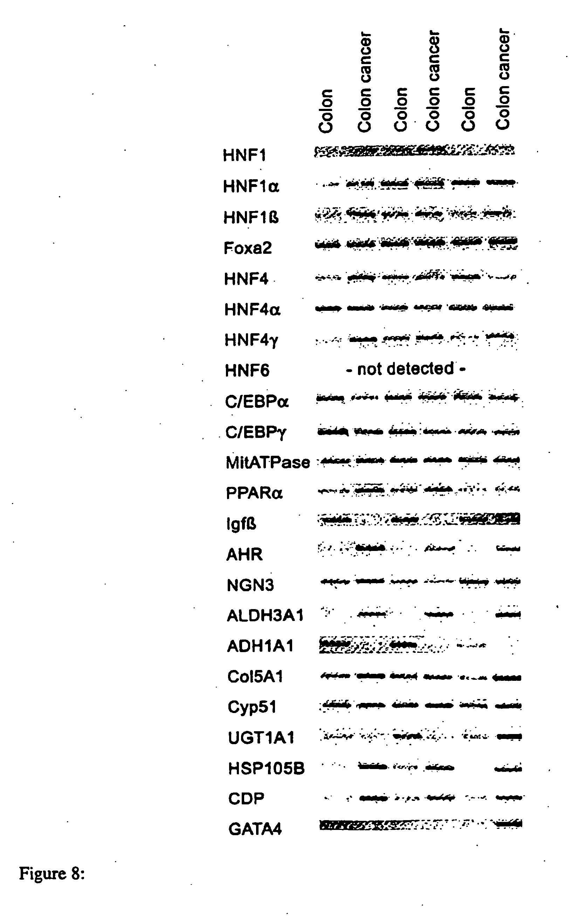

Expression of liver enriched transcription factors was studied in tumour resection material of the colon. This enabled a better understanding of transcript regulation in colorectal liver metastases. Again, tissue material was qualified by histopathology and expression of transcription factor was computed relative to the housekeeping gene mitochondrial ATPase, as described above. No statistical significant difference was obtained when transcript abundance in extracts of healthy colon tissue was compared with RNA extracts of tumour tissue. Strikingly, HNF6 was not expressed in healthy and/or tumour tissue (Fig. 7). This contrasts findings with liver resection material, where HNF6 transcripts were expressed, albeit at different levels. Notably, none of the transcription factors studied by us were significantly changed when healthy and tumourous colon was compared but expression of HNF1, HNF1β, HNF4, HNF4γ, CEBPα, PPARα, IgF1β, AHR and GATA4 was seen to differ amongst individual patients (Fig.8).

With RNA extracts of healthy and tumourous colon expression of genes,
which are considered to be bona fide targets of HNF6, were investigated. Essentially, ADH1A1 was found to be repressed whereas expression of the heat shock proteins HSP105B and HSP90 was increased. This was of no surprise as transcriptional activation of the aforementioned genes is not solely dependent on the proper function of HNF6. Exaggerated expression of heat shock proteins in tumour tissue is frequently observed and known to facilitate oncogenic protein expression.
Expression of NGN3 was studied. Its expression did not differ statistically when healthy tissue and tumour extracts were compared, despite the fact, that NGN3 is a bonafied target of HNF6.

### Western Blotting of HNF6 in healthy human liver and colorectal liver metastases

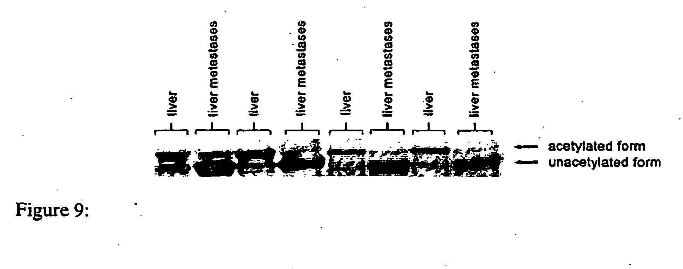

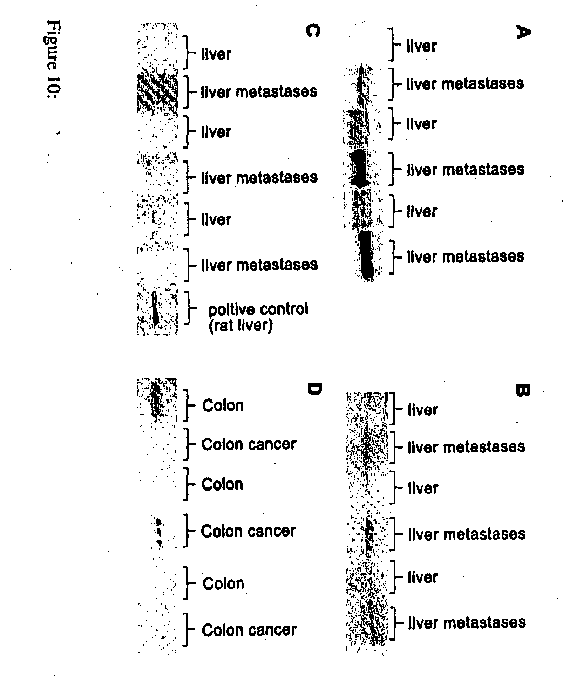

Expression of HNF6 protein in nuclear extracts of healthy liver and colorectal liver metastases was investigated. As shown in Fig. 9 the antibody used detected two immunoreactive bands. Significant differences in the expression level of these bands were observed when nuclear extracts of healthy liver and colorectal liver metastases were compared. Indeed, with nuclear extracts of healthy human liver expression level of the upper immunoreactive bands was strong, whereas with nuclear extracts of colorectal liver metastases a prominent lower immunoreactive band and a faint upper band was observed. Note, the upper band corresponds to the acetylated HNF6, whereas the lower band represents the nonacetylated form. For its known interaction with HNF6 expression of Foxa2 (HNF3β) was investigated in healthy liver and colorectal liver metastases. As shown in Fig. 10A expression of Foxa2 was highly significantly induced in colorectal liver metastases when compared with healthy liver tissue.
Also, induction of HNF1β in colorectal liver metastases was observed (see Fig. 10B). Specifically, the findings according to the inventive work are highly suggestive for differences in the posttranslational modification of the HNF6 protein in healthy and tumourous liver tissue. It has been reported that stability of the HNF6 protein depended on acetylation by the CRB-binding protein coactivator (CBP) with CBP acetylation of the HNF6 protein increasing its steady state levels without influencing nuclear localisation of HNF6.¹⁶ It has also been reported that CBP acetylation influenced nuclear retention of other liver enriched nuclear transcription factors.
Expression of the HNF6 protein differed in healthy and tumourous liver tissue with low level of acetylated HNF6 accounting for the faint upper immunoreactive HNF6 band seen with nuclear extracts from colorectal liver metastases (see Fig. 9). Unfortunately, no commercialantibody is available to probe specifically for the acetylated HNF6 variant, but the study of Rausa et al.¹⁶ clearly demonstrated that transcriptional activity of the HNF6 protein did depend on the CBP acetylation site in the cut domain of this protein. As discussed below, a total of 142 genes targets of HNF6 was studied by gene chip analysis, most of which were significantly repressed, when compared with healthy liver tissue of the same patient. This agrees well with the inventive conclusion of impaired HNF6 DNA binding in colorectal liver metastases. Notably, HNF6 transcript was not detectable in healthy colon or colonic tumour tissue. Hence, no attempts were made to estimate HNF6 protein level in healthy or colonic tumour tissue. As denoted above Foxa2 interacts with HNF6. Therefore Foxa2 expression was investigated in healthy colon and colonic tumours and observed occasionally in healthy or colonic cancer a faint immunoreactive band of which an example is given in Fig. 10D.

Note, very recently the formation of a C/EBPα-HNF6 protein complexes was reported to stimulate HNF6 transcriptional activity.¹⁷ In the inventive research work it was not possible to detect C/EBPa protein in healthy liver or colorectal liver metastases as depicted in Fig. 10C.

### Electrophoretic mobility shift assays

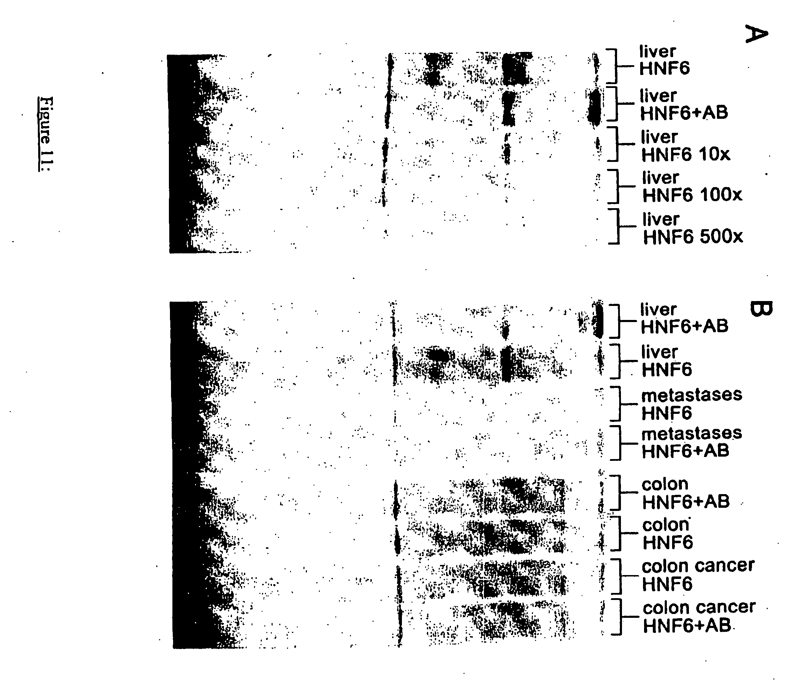

An optimized oligonucleotide probe was employed to investigate DNA binding of HNF6. Specifically, abundant DNA binding was observed with nuclear extracts of healthy liver and specificity for HNF6 by shifting the band with a HNF6 antibody was confirmed. HNF6 DNA binding was further studied in competition assays with unlabeled probe to demonstrate selectivity (Fig. 11A). Strikingly, when nuclear extracts of colorectal liver metastases were used no binding of HNF6 was observed (Fig. 11 B). Likewise, when probed with nuclear extracts isolated from healthy colon no HNF6 band was observed. Thus, DNA binding of HNF6 is only observed in healthy liver tissue. The findings as, inter alia, received according to the invention demonstrate paradoxical expression of HNF6 in colorectal liver metastases as evidenced by gene expression and Western immunoblotting studies (see Fig. 9).

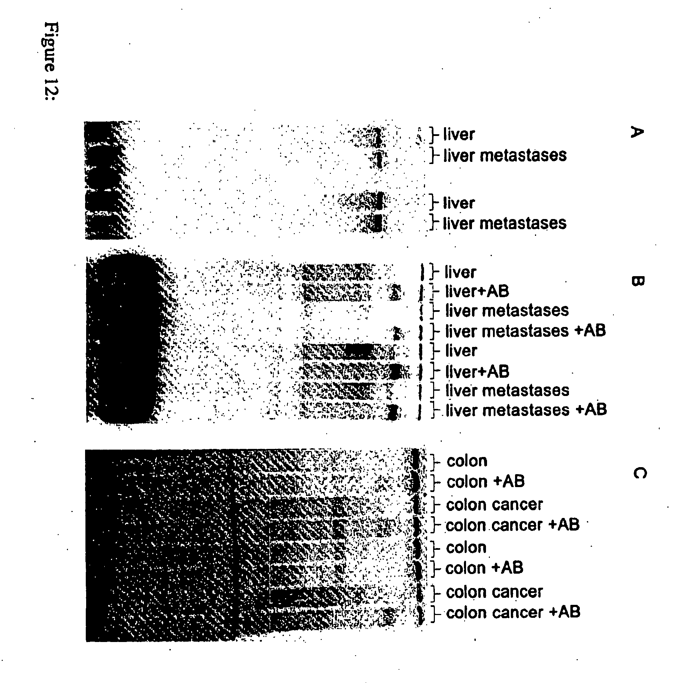

For its well known regulation by HNF6 DNA binding of the NGN3 protein was studied using nuclear extracts of liver and colon. This protein is required for endocrine cell fate and development of intestinal and gastric epithelium. Specifically, HNF6 is an upstream activator of neurogenin 3 and expression of NGN3 depends on HNF6 binding to promoter sites of NGN3. Mice lacking HNF6 display severely reduced NGN3 expression and a reduced number of endocrine cells.¹² NGN3 DNA binding was found not to differ amongst healthy liver and/or colorectal liver metastases (Fig. 12A).

Finally, isoform specificity of HNF6 DNA binding was probed for and published probes to distinguish amongst the different HNF6 variants were used. Oligonucleotide probes optimized for HNF6 binding were employed using promoter sequences of transtyrethin (TTR), hepatic nuclear factor 3 β (=Foxa2), hepatic nuclear factor 4 (HNF4) as well as phosphoenolpyruvat carboxikinase (PEPCK) to differentiate amongst HNF6 isoforms.¹⁸

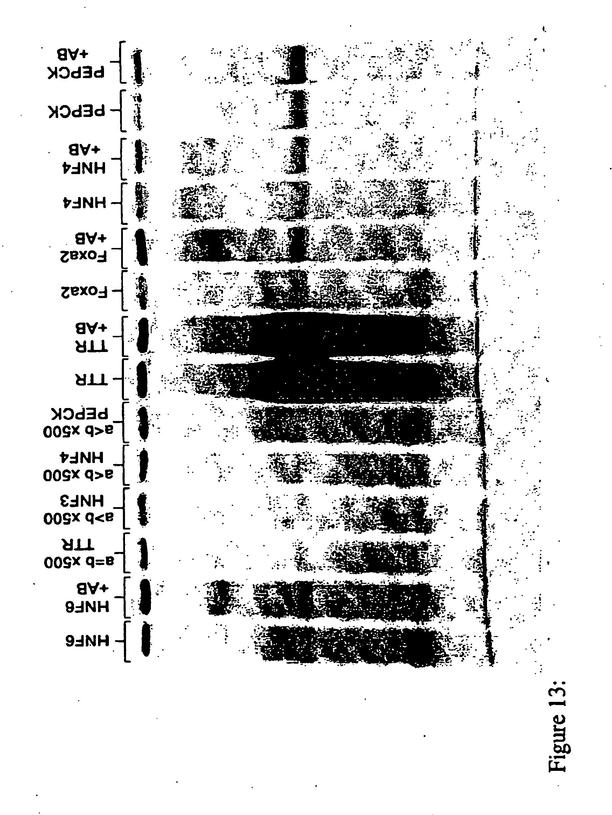

Specifically, binding affinity of HNF6β was reported to be greater for the Foxa2 probe, whereas binding affinity of HNF6α is greater in the case of the HNF4 and PEPCK probe.18 As shown in Figure 13 HNF6 binds to an optimized HNF6 probe (see lane 1). Once again, specificity of binding was confirmed by use of a HNF6 antibody (see lane 2 supershifted band). Then, competition assays with unlabeled probes for TTR, Foxa2, HNF4 and PEPCK were performed to differentiate amongst relative binding affinities of the highly homologous HNF6 a and b isoforms. As can been seen from Figure 13 competition of the HNF6 band was achieved for all but the PEPCK probe. Differences in relative binding affinities for HNF6 isoforms were not determined in this approach. According to the invention, antibodies for distinguishing between these highly homologous isoforms of HNF6 are approriate for realizing this aspect of the invention.

Further, binding of HNF6 to promoter sequences within the TTR, Foxa2, HNF4α and PEPCK gene was studied (see material and methods for the genetic algorithm applied to predict exact location of the HNF6 sites) using nuclear extracts of healthy human liver. Notably, binding of HNF6 to the TTR probe was most abundant. In the case of TTR and Foxa2 binding of HNF6 was confirmed by use of an HNF6 antibody. No HNF6 band could be supershifted with the HNF4a and/or PEPCK probe. In the case of PEPCK results agree well when findings from the competition assay (see lane 6) are compared with HNF6 DNA-binding studies (lane 13+14), whereas findings with the HNF4 probe are less obvious (see lane 5, 11, 12).

Foxa2 DNA binding with nuclear extracts from healthy liver, colorectal liver metastases, healthy colon and colonic tumour was investigated. Essentially, no difference in DNA binding was observed, albeit Foxa2 DNA binding was less abundant with nuclear extracts from healthy or cancerous colonic tissue (see Fig. 12B and 12C).

### Expression of HNF6 target genes

As detailed in the material and methods a total of 24 liver metastases as well as 10 healthy livers were used in the microarray study according to the invention. Expression of HNF6 target genes was specifically probed for. The selection of HNF6 gene targets is based on the study of Odom et al. 2004, who employed chromatin immunoprecipitation followed by DNA/DNA hybridization (CHIPchip assay) to determine HNF6 binding to promoter sequences of the human liver genome.¹¹
In table 5 a selection of the 142 genes targeted by HNF6 is given. The selection is based on major biological functions and included genes coding for general metabolic functions, protein synthesis, transport, receptor, apoptosis and promoter of tumour growth. As shown in table 5 most of the genes targeted by HNF6 were highly significantly repressed, albeit at different levels. It is of considerable importance that three genes involved in promotion of tumour growth were significantly elevated, e.g. chemokine ligand 1, chemokine ligand 3 and fatty acid binding protein.

### DNA mutation analysis of HNF6 CBP binding domain and acetylation sites

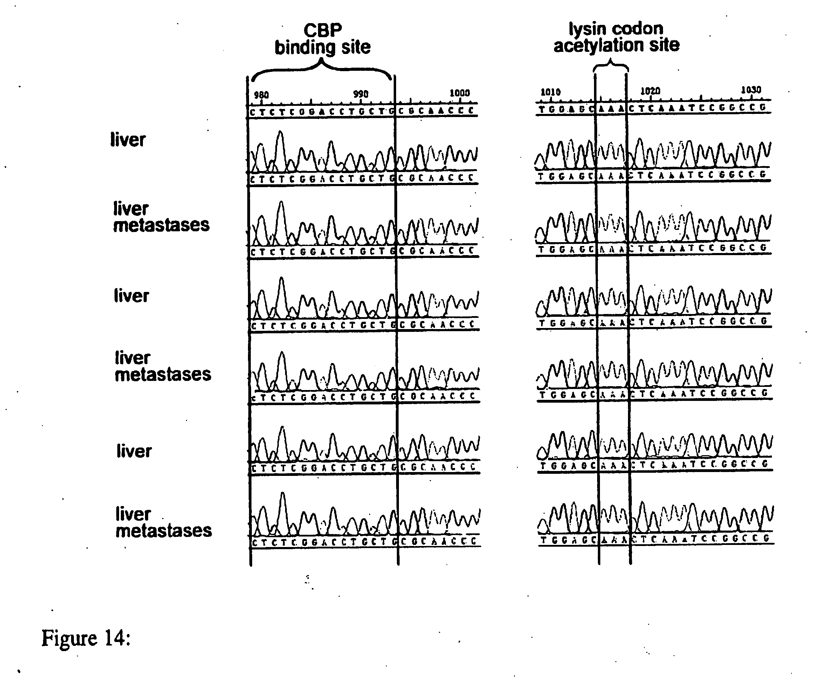

As described above no DNA binding of HNF6 was observed with nuclear extracts of colorectal liver metastases. This prompted the interest in studying sequence variations in the DNA binding domains of HNF6. After PCR amplification the coding sequences of the Cut and homeodomain was studied and amplification products to direct sequencing employing capillary electrophoresis was subjected. No sequence variations were found in the DNA binding domains (Cut and homeodomain) when genomic DNA of healthy human liver and colorectal liver metastases was compared (see Fig. 14). Note, electropherograms of n=3 representative patients showing sequences of genomic DNA extracts of healthy human liver and colorectal liver metastases of a HNF6 acetylation site within the Cut domain are displayed.
Specifically, the codon AAA at position 1015-1017 codes for a lysine. When substituted for arginine (K339R mutant protein) CBP acetylation is abrogated.¹⁶ In human hepatoma cells the mutant HNF6 protein fails to accumulate and is transcriptionally inactive. It is also of considerable importance that such mutant protein cannot be stabilized by inhibiting ubiquitin proteasomal degradation.¹⁶ Thus, abrogation of HNF6 DNA binding cannot be explained by mutations in the Cut and/or homeodomain.

### Hierarchical gene cluster analysis of liver enriched transcription factors and some target genes

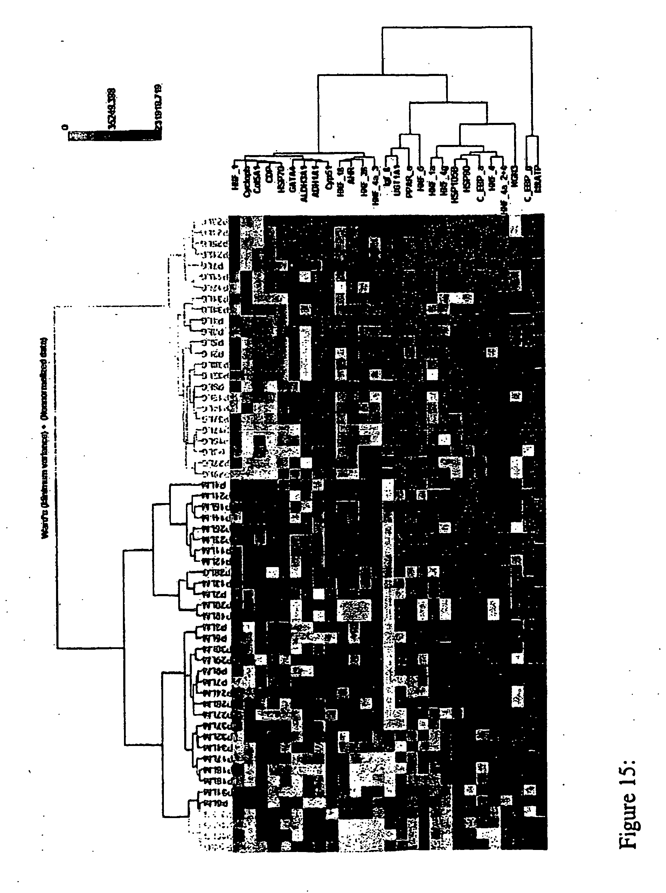

A hierarchical cluster method to group genes on the basics of similarity of their expression was applied. The cluster diagram is shown in Fig. 15 and is based on 783 gene expression results. Despite its complex nature the clustering analysis provided a remarkable order. With a single exception the expression segregates clearly between healthy and cancerous tissue. The dentogram shown on the right hand of Fig. 15 indicates a complex cluster that groups distinct sets of genes with the mitochondrial ATPase (housekeeping gene) being distinctly separated from all other genes.

Groups of distinct sets of genes were observed and this included HNF4 and some of its splice variants, HNF1 and HNF4 (note there is coregulation between HNF1 and HNF4), HSP90 and HSP105, amongst others. Cleanly, gene cluster analysis is useful in identifying potential networks of regulated genes as will be discussed below.
Conclusion: HNF6 protein expression in colorectal liver metastases is paradoxical and driven by the hepatic environment. It is not expressed in healthy or primary colonic cancer. DNA binding of HNF6 is selectively abrogated through lack of posttranslational modification and interaction with Foxa2. Targeting HNF6 may enable mechanism based therapy for colorectal liver metastases by reversing the malignant phenotype.

This study aimed for an improved understanding of liver enriched transcription factor networks in primary tumours of the colon and of colorectal liver metastases. Initially, gene expression of major liver enriched transcription factors as summarized in table 2 was investigated. Patients with suspected or proven familial adenomatous polyposis, which results in colonic cancer due to mutations in the APC gene^{19, 20} were excluded. Amongst the patients the time between resection of primary tumour and colorectal liver metastases was approximately 14 month and did not differ between gender. Of all transcription factors investigated HNF6 and its downstream target genes HNF1β and C/EBP were significantly regulated in colorectal liver metastases. This was unexpected as HNF6 transcript expression was below the limit of detection in healthy tissue or primary tumours of the colon, but expression of the target genes HNF1β and C/EBP's was observed. There was no obvious relationship between HNF6 gene expression and tumour staging although a statistically significant Spearman's correlation coefficient could be determined for HNF6 gene expression in healthy liver as compared with colorectal liver metastases.

The invention provides evidence that anchorage and growth of descendent tumour cells of the primary tumour in a hepatic environment resulted in pleiotropic expression of HNF6. This suggests that an organ specific environment and exposure to tissue specific nutrients and growth factors impacts expression of genes in decadent colonic tumour cells grown in a hepatic environment. Additionally, expression of HNF6 protein and DNA binding to cognate recognition sequences of HNF6 regulated genes were investigated. Remarkably, HNF6 protein expression differed in healthy liver and colorectal liver metastases when nuclear extracts of these tissues were used for Western immunoblotting experiments. HNF6 acetylation was found to be abrogated in colorectal liver metastases. Based on the investigations of Rausa et al. HNF6 protein stability depended on acetylation by the CREB-binding protein coactivator. This explains lack of HNF6 DNA binding which was completely abrogated when nuclear extracts of colorectal liver metastases were used. Foxa2 protein expression was investigated in healthy liver and colorectal liver metastases and expression of Foxa2 was found to be highly induced in nuclear extracts of colorectal liver metastases. Specifically, there is strong evidence for Foxa2 to inhibit HNF6 DNA binding. The protein interaction of HNF6 and Foxa2 results in transcriptional repression of genes targeted by HNF6.²¹ Massive repression of gene expression regulated by HNF6 (see Table 5) was observed. Furthermore, stability of the HNF6 transcription factor depended on acetylation by the CREB-binding coactivator.¹⁶ CBP acetylation is required for HNF6 transcriptional activity. As denoted above, two distinct immunoreactive bands were observed in Western immunoblotting (see Fig. 9) with nuclear extracts of colorectal liver metastases. Certainly, the low level of acetylated HNF6 accounted for the faint upper immunoreactive band, whereas the prominent lower band represents an unacetylated form. In colorectal liver metastases a distinct HNF6 variant could be identified. According to the invention abrogation of HNF6 DNA binding to genes targeted by this factor is found to be the result of an inhibitory protein interaction with the transcription factor Foxa2. Notably, Foxa2 was strongly induced in colorectal liver metastases as shown in Fig. 10A. There is evidence for HNF6 to serve as a coactivator protein to enhance Foxa2 transcription. The approach performed according to the invention demonstrates binding of HNF6 to promoter sequences of Foxa2 (see Fig. 13, lane 9 and 10) with nuclear extracts from healthy liver and observed variable Foxa2 binding with nuclear extracts form colorectal liver metastases. Likewise, Foxa2 protein expression was variable in healthy and tumourous colonic tissue (see Fig. 10B). Further evidence stems from RT-PCR experiments where repression of transcripts of HNF6 regulated genes was an overwhelming feature of metastatic liver growth. Additionally, by gene chip analysis genome wide transcript abundance in healthy liver and liver metastases were investigated using the Affymetrix platform. The result of this investigation is the subject of a separate publication. Nonetheless, a total of 142 gene targets of HNF6 were identified, of which a small proportion is given in table 5. Most of the HNF6 regulated genes were repressed even though regulators of tumour promotion were induced. Additionally, sequence variations in the DNA binding domain of HNF6 were investigated but no mutations could be identified.

Taken collectively expression of HNF6 is exceptional for colorectal liver metastases. Neither healthy nor primary colonic tumour tissue expresses HNF6. The work according to the invention provides conclusive evidence for a HNF6 variant to be strongly expressed in colorectal liver metastases but the protein is unable to bind to HNF6 recognition sequences. Likely, abrogation of HNF6 DNA binding is due to interaction with Foxa2, which was found to be highly induced in colorectal liver metastases.

Specifically, hepatocyte nuclear factors play an essential role in determining cellular differentiation. There is a complex network of hepatocyte nuclear transcription factors acting in concert to enable the many metabolic functions of hepatocytes. These nuclear factors function in a networked environment and bind to recognition sequences of targeted genes, therefore providing regulatory chains where one hepatic nuclear factor activates another one. Such regulatory loops are of particular importance in the onset and progression of disease. For instance, HNF6 binds to cognate recognition sequences of HNF4a to regulate its expression.²² Recent evidence suggests loss of HNF4α expression to be an important determinant of HCC progression.²³ HNF4α binds to the A-site within the HNF1α promoter to regulate its activity.
The hepatocyte nuclear factor network determines liver specific gene expression as recently reviewed by us.^{8, 9} Unlike the study of Lazarevich et al, who studied expression of liver enriched transcription factors in slow and fast growing hepatocellular carcinomas, most of the liver enriched transcription factors studied by us were expressed in secondary malignancies of the liver, albeit at different levels. Indeed, expression of HNF6 in colorectal liver metastases is paradoxical and is a consequence of the nutrient and growth factor environment of the liver.

This is the first report to demonstrate the gene environment interaction in metastatic disease. The findings according to te invention do well translate to novel therapies, as HNF6 networking and transcriptional regulation is abrogated in colorectal liver metastases. Likely, DNA binding of HNF6 impacts regulation of liver specific gene expression and by implication disease phenotype.
The relationship between HNF6 and Foxa2 is controversial. In the study of Rausa et al. HNF6 functions as a coactivator protein to potentiate the transcriptional activity of Foxa2 ²¹, but in the recent study of Rubins et al HNF6 function is largely independent of Foxa2.²⁴
According to one aspect of the invention, Foxa2 regulation is now demonstrated to be independent of HNF6 and massive induction of Foxa2 protein is observed in colorectal liver metastases.
As discussed above, HNF6 DNA binding is absent in healthy and colonic tumour tissue. It is also absent in colorectal liver metastases, but HNF6 DNA binding can be clearly demonstrated for healthy liver tissue of the same patient.

This study is the first report on the importance of liver enriched transcription factors in secondary liver malignancies. Of all transcription factors investigated HNF6 appears to play a pivotal role. Its' gene and protein expression was surprisingly found to be driven by the nutrient supply of the liver. HNF6 binding to DNA is demonstrated to be selectively abrogated. This points to a molecular mechanism by which Foxa2 inhibits HNF6 DNA binding.

Experiments were performed using, inter alia, the following methods and means.

### Patients characteristics

Approval for the use of tissue material was obtained from the ethics committee of the Medical School of Hannover, Germany (study number 3416 of the Medical School of Hanover). All patients participating in this study gave written informed consent and were fully aware of the aims of the study. In addition, patients with either suspected or proven familial adenomatous polyposis (FAP) were deliberately excluded. Specifically this autosomal dominant disorder results in colorectal cancer in early stages of adult live as a result of mutations in the APC gene. Table 1 provides a synoptic overview of patient's characteristics including age, gender, primary diagnosis and tumour staging as well as times span between primary tumour and colorectal liver metastases. Notably, the patients reported in table 1 received either surgery of the colon (e.g. primary tumour resection) or colorectal liver metastases, but none of the patients received surgery for colon and liver tumours simultaneously.

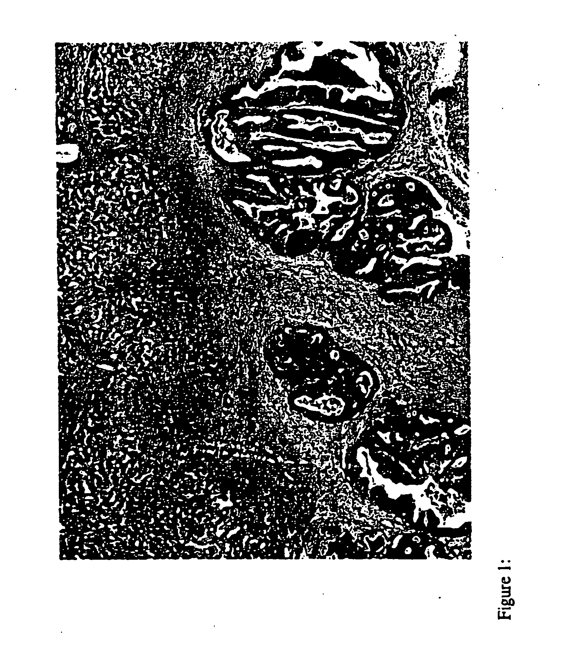

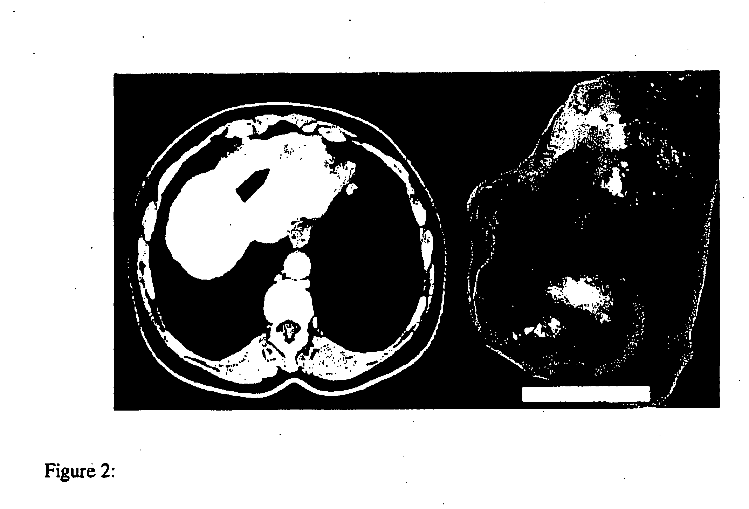

The median age of patients with liver metastases was 63 years (n=29) and the distribution of gender was 45% males and 55% females. Based on histological examination of resected material, all patients were diagnosed with medium or poorly differentiated liver tumours (Fig. 1). Patients had no family history of malignant diseases or genetic disorders, e.g. familiarly adenomatous polyposis coli In addition, clinical chemistry parameters of liver function were within normal range and no extrahepatic metastatic growth was observed, as evidenced by preoperative imaging (e.g. CT-scan, MRI, ultrasound, an example is given in Fig. 2). The median disease-free time was 1.2 years after resection of the primary colon tumour and ranged from 0 to 8 years.

In the patient cohort of colon tumours (n=16) the median age was 64 years and the distribution of gender was 63% male and 37% female. Patients were diagnosed with primary colorectal cancer. This was confirmed by histopathology.
Noteworthy, preparation of tissue material from liver or colon was such that biopsies were either within the tumour or within normal tissue, as detailed below.

### Explanted human material

The primary colonic tumours and/or the colorectal liver metastases were removed by standard surgical procedures. All surgical specimens were subjected to histopathology. Excised healthy and tumourous tissue was shock-frozen in liquid nitrogen and stored at -80°C until analyzed.

### RNA isolation and cDNA synthesis

RNA was isolated form tissue samples using the RNeasy Mini Kid (Quiagen) according to the manufacturer's recommendation. Quality and quantity of isolated RNA were checked by capillary electrophoresis (Bioanalyzer 2100, Agilent Technologies) following the manufacturer's instructions or by gelelectrophoresis. 2µg total RNA from each sample was used for reverse transcription (RT). RNA and random pimer (Promega, Mannheim, Germany) were preheated for 10 min at 70°C and then chilled on ice for 2 min. A total of 5 x RT-avian myoblastosis virus (AMV) buffer (Promega), dNTP's (10mM), RNAsin, AMV-RT (avian myeloblastosis virus-reverse transcriptase) (all Promega) and DEPC-H2O were added to a final volume of 20 µl. Reverse transcription was carried out for 60 min at 42°C and was stopped by heating to 95°C for 5 min. The resulting cDNA was frozen at -20°C until additional experimentation.

### Thermocycler RT-PCR

Primer design was done with the program Primer 3 (http://frodo.wi.mit.edu/cgibin/primer3/primer3_www.cgi). Cross-reaction of primers with the genes was excluded by comparison of the sequence of interest with a database (Blast 2.2, US National Centre for Biotechnology Information). PCR reactions were undertaken with a 20 µl reaction mixture containing HotStarTaq Master Mix (Qiagen, Hilden, Germany), DEPC, 1 µl c DNA and 1,0 µM concentration of the 3'- and 5'-specific oligomers (synthesized by Invitrogen, Hilten, Germany). PCR reactions were carried out on a thermal cycler (T3, Biometra). Detailed oligonucleotide sequence information and the PCR amplification protocol is given in table 2. DNA contamination was checked for by direct amplification of RNA extracts before conversion to cDNA. Contamination of RNA extracts with genomic DNA could be excluded. PCR reactions were done within the linear range of amplification, and amplification products were separated using 1.5% agarose gel and stained with ethidium bromide. Gels were photographed on a transilluminator (Kodak Image Station 440), and amplicons were quantified using the Kodak 1 D 3.5 network software.

### Western Blotting experiments

Western immunoblotting was done as follows: Total protein (100 µg) or nuclear protein (30 µg) extracts from healthy or tumourous liver probes were denaturated at 95 °C for 5 min, followed by sodium dodecyl sulphate polyacrylamide gel electrophoresis (SDS-PAGE) on 12 % polyacrylamide gels and blotted onto a polyvinylidene difluoride membrane (NEN,

Dreieich, Germany) at 350 mA for 2 h in a buffer containing 400 mM glycine, 50 mM Tris (pH 8.3). Nonspecific binding sites were blocked with Rotiblock (Roth, Germany) in 1x TBS buffer. After electroblotting of proteins, membranes were incubated with polyclonal antibodies for HNF6 (kind gift of Dr. R.H. Costa, Chicago, Illinois, USA) for one hour and washed 3-times with 1 x TBS buffer containing 0.1 % Tween-20 (Roth, Germany). Subsequently, the membranes were incubated with a 1:5000 diluted anti a-rabbit) antibody (Chemicon, Hofheim, Germany) for 1 h at room temperature followed by 3 successive washes with 1x TBS buffer containing 0.1% Tween-20 (Roth, Germany). Immunoreactive proteins were visualized with a chemiluminescence reagent kit (NEN, Dreieich, Germany) according to the manufacturers instructions, and bands were scanned with the Kodak Image Station CF 440 and analysed using the Kodak 1 D 3.5 imaging software (Eastman Kodak Company, USA).

### Preparation of nuclear extracts

For the preparation of nuclear extracts the protocol of Gorski et al was employed with modifications.¹⁰ Following explanation tissue specimens were place on ice-cold PPB containing buffer and swiftly transported to the laboratory (e.g. time of explantation to tissue preparation was approximately 30 minutes). After determining the weight tissue materials were cut into small pieces and placed into a vessel containing buffer HP1 which consisted of 25 x Complete™ , 1 M DTT, 0.5 M EDTA, pH 8.01 M b-glycero-phosphat, glycerin1 M, Hepes, pH 7.61 M KCI, 200 mM Na₃VO₄, 0.1 M spermine, 3.44 M spermidine, sucrose bidest H₂O. Samples were homogenized with a Ultra-Turrax (Janke&Kunkel, IKA Labortechnik, Germany). Then, the suspension was transferred to a 15ml hand-potter homogenizer (Wheaton, USA) to achieve a homogenous suspension. Thereafter the suspension was transferred into SW28 tubes (UltraClear Beckmann, USA) and the volume was adjusted and centrifuged at 24000 rpm at 2°C in a Beckmann L7-55 ultracentrifuge for one hour. After centrifugation nuclear pellets were isolated and resuspended in buffer HP1 containing glycerine at a concentration of 19 volume percent. Once again the resuspended pellet was homogenized in a hand-potter (Wheaton, USA). Thereafter the solution containing nuclear extracts was placed into SW28 tubes and centrifuged at 24000 rpm at 2°C in a Beckmann L7-55 centrifuge for a total of one hour. Finally the supernatant was discarded and the pellet was suspended in 1-2 ml lysis buffer ontaining 25 x Complete^{™}, 1 M DTT, 0.5 M EDTA, pH 8.0, 1 M b-glycerophosphat , glycerine, 1 M Hepes, pH 7,6, 1 M KCI, 1 M MgCl₂, 200 mM Na₃HPO₄, bidest H₂O. Once again this suspension was homogenized in a hand-potter (Wheaton, USA) and nuclei were inspected microscopically. In addition DNA amount was determined spectrophotometry by determining the ratio of optical densities 280 over 260 nm. The DNA concentration was adjusted to 0.5 mg DNA/ml suspension. Then 4M ammonium sulphate was added (1/10 of the final volume) and samples were placed on ice for 30 minutes. Thereafter, the solution was placed into a Ti70.1 tube (Beckmann, USA) and centrifuged at 40.000 rpm at 2°C for one hour. The resultant supernatant was carefully removed and the volume was determined. Precipitation of nuclear extracts was achieved by addition of 0.3g anhydrous ammonium sulphate per ml supernatant and samples were placed on ice for 45-60 minutes. Finally the ice-cold solutions were placed into Ti70.1 tubes (Beckmann, USA) and centrifuged at 40000 rpm at 2°C for one hour. Then the supernatant was discarded and the pellet was taken up in a dialysis buffer containing 1 M DTT, 0.5 M EDTA, pH 8.0, glycerin, 1 M Hepes, pH 7.6, 1 M KCI, bidest H₂O. A final DNA concentration of 10 µg/ml was adjusted and the samples were placed on ice for 30-6.0 minutes. Notably, these samples were placed into a Slide a Lyzer Dialysis cassette (Pierce, USA) and stored at 4°C. Following 2 hours of dialysis the lysis buffer was replaced and the samples was once again dialysed for a further 2 hours. Then, the samples were taken from the cassette and placed into 1.5 ml Eppendorf vessels and centrifuged at 14000 rpm at 4°C for 5 minutes. The concentration of resultant nuclear proteins was determined by the method of Smith and the remaining nuclear proteins was stored at -80°C to await further analysis.

### Annealing of synthetic oligonucleotides and [32P] labeling.

Oligonucleotides representing a high affinity consensus HNF6, NGN3, HNF3, HNF4, TTR and PEPCK binding site were chosen. For sequence information see Table 3. Oligonucleotides were annealed at a concentration of 19.2 pM µl⁻¹ in 200 mM Tris (pH 7.6), 100 mM MgCl₂ and 500 mM NaCl at 80 °C for 10 min and then cooled slowly to room temperature overnight and were stored at 4 °C. Annealed oligonucleotides were diluted to 1:10 in Tris-EDTA buffer (1 mM EDTA, 10 mM Tris, pH 8.0) and labeled using [32P] ATP (Amersham Biosciences Europe GmbH, Freiburg, Germany, 250 µCi, 3,000 Ci mM⁻¹) and T4 polynucleotide kinase (New England Biolabs GmbH, Frankfurt am Main, Germany). Endlabeled probes were separated from unincorporated [32P] ATP with a Microspin G-25 Column (Amersham Biosciences Europe GmbH, Freiburg, Germany) and eluted in a final volume of 100 µL.

### Electrophoretic Mobility Shift Assay (EMSA)

The procedure for EMSA was adapted from a previously described method. Briefly, 7.5 µg of nuclear extract were incubated with the binding buffer consisting of 25 mM HEPES (pH 7.6), 5 mM MgCl₂, 34 mM KCl, 2 mM DTT. 2 mM Pefablock (Roche Diagnostics GmbH, Mannheim, Germany), 0.5 µL aprotinin (2.2 mg · mL⁻¹, Sigma-Aldrich Chemie GmbH, Taufkirchen, Germany), 50 ng poly (dl-dC) and 80 ng bovine serum albumin (PAA Laboratories GmbH, Cölbe, Germany). The binding reaction was carried out for 20 min on ice and free DNA and DNA-protein complexes were resolved on a 6 % polyacrylamide gel. Competition studies were done by adding a 10-fold excess of unlabeled oligonucleotides to the reaction mix. For supershift studies a specific HNF6 antibody (Santa Cruz Biotechnology Inc., Heidelberg, Germany) were added to the reaction mix 10 min before addition of the labeled probe. Gels were blotted to Whatman 3 MM paper, dried under vacuum, exposed to imaging screens (Imaging Screen-K, Bio-Rad Laboratories GmbH, München, Germany) for autoradiography overnight at room temperature and analysed using a phosphor imaging system (Molecular Imager FX pro plus; Bio-Rad Laboratories GmbH, München, Germany) and the Quantitiy One Version 4.2.2 software (Bio-Rad Laboratories GmbH, München, Germany).

### Microarray experiments

Global gene expression analyses were done with n=24 colorectal liver metastases and n=10 healthy livers as detailed below:

### RNA isolation and production of copy RNA

The cRNA samples were prepared following the Affymetrix Gene Chip^{®} Expression Analysis Technical Manual (Santa Clara, CA, USA). Briefly, total RNA was isolated from frozen tissue using QIAGEN's RNeasy total isolation procedure. A second cleanup of isolated RNA was performed using the same RNA isolation kit. In all, 10 µg of total RNA was used for thesynthesis of double-stranded cDNA with Superscript II RT an other reagents from Invitrogen Life Technologies. HPLC-purified T7-(dT)₂₄ (GenSet SA) was used as a primer. After cleanup, double stranded cDNA was used for the synthesis of biotin-labelled cRNA (Enzo^{®} BioArray High Yield RNA Transcript Labeling Kit, Affymetrix). cRNA purified with Rneasy spin columns from Qiagen was cleaved into fragments of 35-200 bases by metal-induced hydrolysis.

### Array hybridization and scanning

A measure of 10 µg of biotinylated fragmented cRNA was hybridized onto the HG U95Av2 Array which contains approximately 10.000 full-length genes.
The hybridized, washed and coloured arrays were scanned using the Agilent Gene Array^{®} Scanner. Scanned image files were visually inspected for artefacts and then analysed, each being scaled to an all probe set intensity of 150 for comparison between chips. The Affymetrix Microarray Suite (version 5.0) was used to control the fluidics station and the scanner, to capture probe array data and to analyse hybridization intensity data. Default parameters provided in the Affymetrix data analysis software were applied in running of analysis.

### Data analysis

The hybridization values for each gene probe presented on the array with a set of 16 perfect and mismatch oligonucleotide pairs were calculated with the Affymetrix Microarry Suite 5.0 Software, using the manufacture's statistical algorithm. The results were reported as numeric expression values - signal intensities and absolute information - detection calls "Present" or "Absent" produced by two independent algorithms. The results of a single comparison analysis between two different arrays were reported for each gene as signal logarithm ratio (log₂ratio) and a change called "Increase" or "Decrease". Multiple data from replicate samples were evaluated and compared using statistical analysis with the Affymetrix Data Mining Tool 3.0 (DMT). The average and standard deviation statistics within Affymetrix DMT was used to summarize the expression level (the signal values) for each transcript across the replicates.
The unpaired one-sided T-test converting P-value to a two-sided P-value was used to determine the level between sets of colorectal liver metastases and healthy liver tissue, with the P-value cutoff determined as 0.05. Besides, only those genes that were detected (had call "Present") in all samples of colorectal liver metastases and healthy liver tissue were taken into consideration as differentially expressed. Fold-chance values were calculated as the ratio of the average expression level for each gene between two tissue sets. Comparison ranking analysis was additionally employed to study the concordance of gene expression changes in pair wise comparisons of tumour samples with healthy liver tissue. The results are shown as % of "Increase" or "Decrease" calls in individual comparisons.

### HNF6 DNA sequence analysis

DNA Isolation Genomic DNA from healthy human liver and colorectal liver metastases was isolated with the NucleoSpinTissue Kit (Macherey-Nagel, Düren, Germany) according to the manufacturer's instructions. The quality and quantity of isolated genomic DNA was checked on ethidiumbromide stained 1% agarose gel using known lambda DNA concentration (Amersham, Freiburg, Germany) as standard.

### PCR Amplification

PCR primers were designed with the publicly available PRIMER3 (http://frodo.wi.mit.edu/cgi-bin/primer3/primer3_www.cgi) software and published sequence information of HNF6 according to GenBank (NCBI) entry. The DNA binding domains (Cut and Homeodomain) were specifically amplified (see table 4 for the 3' and 5' specific primers) using oligonucleotide synthesized by Invitrogen (Hilten, Germany). A standard PCR reaction consisted of about 20 ng of genomic DNA, 2.5 µl of PCR buffer (10x), 0.2 µl of *Taq* polymerase (5 U/µl), 0.5 µl dNTPs (10mM), 0.5 µl of each primer pair (10 pmol/µl) adjusted to a volume of 25 µl with distilled water. Typical PCR conditions consisted of an initial denaturation of 95°C for 15 min; followed by 34 cycles of 94°C 10 s denaturation, 60°C 30 s annealing, and 68°C 2 min elongation; and a final elongation at 68°C for 10 min. PCR reactions were carried out on Biometra (Germany) thermocyclers. PCR products were analyzed on GelDoc 2000 (Bio-Rad), using ethidium bromide-stained 1% agarose gels, arid a 1 kb-plus ladder as size marker (Invitrogen). A negative control (water only) was included for PCR amplification.

### Sequencing of DNA binding domains

Mutations were searched for double-strand direct sequencing using gene-specific primers. Amplified fragments were purified with PCR clean-up kits according to manufacturer's protocol (OlAquick PCR Purification Kit, Qiagen) and subjected to cycle sequencing with BigDyeTerminator v3.1 Kit following the manufacturer's procedure, and injected to an ABI 3100 Genetic Analyzer (Applied Biosystems, Darmstadt, Germany). Sequences were analyzed for nucleotide changes using appropriate programs (SeqScape, Applied Biosystems). Sequence for HNF6 as published at GenBank (NCBI) was used as reference.

### Hierarchical gene cluster analysis

Hierarchial gene cluster analysis was done to the Ward's minimum variance algorithm. Gene expressions are given as signal intensities obtained from ethidium bromide stained images.
The genes are arranged as ordered by the clustering algorithm. The colour image is proportional to transcript abundances, with dark green colours representing less abundant and red colours representing more abundant mRNA transcripts.

It was surprisingly found, that many of the liver enriched transcription factors were expressed in primary tumours of the colon and in colorectal liver metastases, but for example HNF6 gene and protein expression was confined to liver metastatic growth. Though abundantly expressed, HNF6 was unable to bind to promotor sequences of targeted genes. Genomic sequencing did not indicate variations in the binding domains of HNF6, but Western blotting of HNF6 identified unacetylated HNF6 as a hallmark of colorectal liver metastases. Because of its known interaction with HNF6 expression of Foxa2 was investigated and found to be specifically induced in colorectal liver metastases. Furthermore, expression of 142 genes targeted by HNF6 was studied by gene chip analysis and found to be mostly repressed except for tumour growth.

In the research work leading to the invention, it has now been shown that among all liver-specific transcription factors examined, the acetylation of the hepatic nuclear factor 6 (HNF6) was disturbed, with the consequence that non-hepatic tissue (= thus formed by intestinal cancer cells) successfully grew in the liver and infiltrated the liver, increasingly.
The patient increasingly suffers from a loss of functional liver tissue, thus leading to an uncontrollable metabolism and to the inability to manage normal liver-specific and/or organo specific metabolic processes. The evolving liver insufficiency results in a tumor cachexia, wherein the patient is not able to keep normal organ functions going, anymore, because of metabolic disturbances.

The mortality due to metastases of the liver is very high with less than 40 % probability of survival after 5 years. At present, a chemotherapy is not accomplished along/in combination with a RO resection (= removal of the tumor from the healthy tissue: After detection of metastases by imaging procedures (e.g. CT, MR) a resection of the tumor in the healthy tissue is aimed), although the rate of reciditivs is high, i.e. more than 60 % of the patients develop reciditive metastases.

### Literature

1. Jemal A, Murray T. Ward E, Samuels A. Tiwari RC, Ghafoor A. Feuer EJ. Thun MJ. Cancer statistics, 2005. CA Cancer J Clin 2005;55:10-30.
2. van de Velde CJ. Treatment of liver metastases of colorectal cancer. Ann Oncol 2005;16 Suppl 2:ii144-149.
3. Jain S, Sacchi M. Vrachnos P, Lygidakis NJ, Andriopoulou E. Recent advances in the treatment of colorectal liver metastases. Hepatogastroenterology 2005;52:1567-1584.
4. Lee CS, Friedman JR, Fulmer JT, Kaestner KH. The initiation of liver development is dependent on Foxa transcription factors. Nature 2005;435:944-947.
5. Lemaigre F, Zaret KS. Liver development update: new embryo models, cell lineage control, and morphogenesis. Curr Opin Genet Dev 2004;14:582-590.
6. Parviz F, Matullo C, Garrison WD, Savatski L. Adamson JW, Ning G, Kaestner KH, Rossi JM, Zaret KS, Duncan SA. Hepatocyte nuclear factor 4alpha controls the development of a hepatic epithelium and liver morphogenesis. Nat Genet 2003;34:292-296.
7. Clotman F, Lannoy VJ; Reber M. Cereghini S. Cassiman D, Jacquemin P. Roskams T, Rousseau GG, Lemaigre FP. The onecut transcription factor HNF6 is required for normal development of the biliary tract. Development 2002;129:1819-1828.
8. Schrem H, Klempnauer J, Borlak J. Liver-enriched transcription factors in liver function and development. Part I: the hepatocyte nuclear factor network and liverspecific gene expression. Pharmacol Rev 2002;54:129-158.
9. Schrem H, Klempnauer J, Borlak J. Liver-enriched transcription factors in liver function and development. Part II: the C/EBPs and D site-binding protein in cell cycle control, carcinogenesis, circadian gene regulation, liver regeneration, apoptosis, and liver-specific gene regulation. Pharmacol Rev 2004;56:291-330.
10. Gorski K, Carneiro M, Schibler U. Tissue-specific in vitro transcription from the mouse albumin promoter. Cell 1986;47:767-776.
11. Odom DT, Zizlsperger N, Gordon DB, Bell GW. Rinaldi NJ. Murray HL, Volkert TL, Schreiber J, Rolfe PA, Gifford DK, Fraenkel E, Bell GI, Young RA. Control of pancreas and liver gene expression by HNF transcription factors. Science 2004;303:1378-1381.
12. Jacquemin P, Durviaux SM, Jensen J, Godfraind C, Gradwohl G, Guillemot F, Madsen OD, Carmeliet P, Dewerchin M, Collen D, Rousseau GG, Lemaigre FP. Transcription factor hepatocyte nuclear factor 6 regulates pancreatic endocrine cell differentiation and controls expression of the proendocrine gene ngn3. Mol Cell Biol 2000;20:4445-4454.
13. Jenny M, Uhl C, Roche C, Duluc I, Guillermin V, Guillemot F, Jensen J, Kedinger M, Gradwohl G. Neurogenin3 is differentially required for endocrine cell fate specification in the intestinal and gastric epithelium. Embo J 2002;21:6338-6347.
14. Whitesell L, Lindquist SL. HSP90 and the chaperoning of cancer. Nat Rev Cancer 2005;5:761-772.
15. Akiyama Y, Watkins N, Suzuki H, Jair KW, van Engeland M, Esteller M, Sakai H, Ren CY, Yuasa Y, Herman JG, Baylin SB. GATA-4 and GATA-5 transcription factor genes and potential downstream antitumor target genes are epigenetically silenced in colorectal and gastric cancer. Mol Cell Biol 2003;23:8429-8439.
16. Rausa FM, 3rd, Hughes DE, Costa RH. Stability of the hepatocyte nuclear factor 6 transcription factor requires acetylation by the CREB-binding protein coactivator. J Biol Chem 2004;279:43070-43076.
17. Yoshida Y, Hughes DE, Rausa FM, 3rd, Kim IM, Tan Y, Darlington GJ, Costa RH. C/EBPalpha and HNF6 protein complex formation stimulates HNF6-dependent transcription by CBP coactivator recruitment in HepG2 cells. Hepatology 2006;43:276-286.
18. Lannoy VJ, Burglin TR, Rousseau GG, Lemaigre FP. Isoforms of hepatocyte nuclear factor-6 differ in DNA-binding properties, contain a bifunctional homeodomain, and define the new ONECUT class of homeodomain proteins. J Biol Chem 1998;273:13552-13562.
19. Jo WS, Chung DC. Genetics of hereditary colorectal cancer. Semin Oncol 2005;32:11-23.
20. Baglioni S, Genuardi M. Simple and complex genetics of colorectal cancer susceptibitity. Am J Med Genet C Semin Med Genet 2004;129:35-43.
21. Rausa FM, Tan Y, Costa RH. Association between hepatocyte nuclear factor 6 (HNF-6) and FoxA2 DNA binding domains stimulates FoxA2 transcriptional activity but inhibits HNF-6 DNA binding. Mol Cell Biol 2003;23:437-449.
22. Briancon N, Bailly A, Clotman F, Jacquemin P, Lemaigre FP, Weiss MC. Expression of the alpha7 isoform of hepatocyte nuclear factor (HNF) 4 is activated by HNF6/OC-2 and HNF1 and repressed by HNF4alpha1 in the liver. J Biol Chem 2004;279:33398-33408.
23. Lazarevich NL, Cheremnova OA, Varga EV, Ovchinnikov DA, Kudrjavtseva El, Morozova OV, Fleishman DI, Engelhardt NV, Duncan SA. Progression of HCC in mice is associated with a downregulation in the expression of hepatocyte nuclear factors. Hepatology 2004:39:1038-1047.
24. Rubins NE, Friedman JR, Le PP, Zhang L, Brestelli J, Kaestner KH. Transcriptional networks in the liver: hepatocyte nuclear factor 6 function is largely independent of Foxa2. Mol Cell Biol 2005:25:7069-7077.

**Table 1: Patient characteristics (A: liver; B: colon)**

| A | | | | | | | |
|---|---|---|---|---|---|---|---|
| ID | sex | age | primary tumour location | | | UICC classification of primary tumour | time to metatases (month) |
| P2 | m | 65 | sigmoid | | | I | 14 |
| P3 | f | 70 | colon ascendens | | | II | 99 |
| P4 | f | 66 | sigmoid | | | IV | 169 |
| P5 | f | 65 | sigmoid | | | III | 15 |
| P6 | f | 75 | rectum | | | III | 28 |
| P7 | m | 73 | rectum | | | IV | 3 |
| P9 | m | 70 | colon ascendens | | | IV | 9 |
| P11 | f | 57 | sigmoid | | | III | 4 |
| P12 | m | 45 | rectum | | | III | 34 |
| P13 | m | 62 | sigmoid | | | I | 52 |
| P14 | m | 71 | rectum | | | I | 13 |
| P15 | m | 47 | rectum | | | III | 5 |
| P16 | m | 72 | rectum | | | II | 24 |
| P17 | f | 63 | rectum | | | III | 46 |
| P18 | m | 73 | rectum | | | II | 25 |
| P19 | f | 64 | colon ascendens | | | II | 14 |
| P 20 | m | 50 | rectum | | | III | 11 |
| P 21 | f | 74 | rectum | | | IV | 3 |
| P 23 | m | 69 | rectum | | | II | 30 |
| P 24 | f | 56 | sigmoid | | | III | 11 |
| P 25 | f | 70 | rectum | | | IV | 7 |
| P 27 | f | 51 | rectum | | | I | 51 |
| P 28 | f | 63 | rectum | | | IV | 7 |
| P 29 | f | 40 | sigmoid | | | IV | 14 |
| P 30 | m | 43 | colon ascendens | | | III | 12 |
| P 31 | f | 49 | rectum | | | II | 19 |
| P 32 | f | 62 | coecal | | | IV | 6 |
| P 34 | f | 61 | sigmoid | | | IV | 0 |
| P 37 | m | 63 | sigmoid | | | III | 31 |
| | | | | | | | |

| B: | | | | | | | |
|---|---|---|---|---|---|---|---|
| ID | | | sex | age | localisation | TNM classification | UICC classification |
| CN2/ CP2 | | | m | 64 | rectum | pT2pN0M0 G2 | I |
| CN3/ CP3 | | | F | 74 | transverse | pT2pN1pM1 G2 | IV |
| CN7/ CP7 | | | f | 51 | ascendens | pT3pN2M0 G2 | III |
| CN8 / CP8 | | | m | 81 | sigmoid | pT3pN0M0 G3 | II |
| CN9/CP9 | | | f | 63 | sigmoid | pT2pN1M0 G2 | III |
| CN10 / CP10 | | | m | 81 | rectum | pT3pN1M0 G2 | III |
| CN11 / CP11 | | | f | 49 | ascendens | pT3pN2M1 G2 | IV |
| CN15/CP15 | | | m | 73 | sigmoid | pT4pN0M0 G3 | II |
| CN16/CP16 | | | m | 72 | rectum | pT2pN0M0 G2 | I |
| CN17/CP17 | | | m | 44 | rectum | pT2pN1M1 G2 | IV |
| CN18/CP18 | | | f | 67 | rectum | pT3pN1M1 G2 | IV |
| CN19/CP19 | | | m | 61 | rectum | p77pN2M1 G2 | IV |
| CN20/CP20 | | | m | 56 | rectum | pT3pN0M0G2 | II |
| CN21/CP21 | | | m | 61 | sigmoid | pT3pN2M1 G2 | IV |
| P34 | | | f | 61 | sigmoid | pT4pN1M1G2 | IV |
| P38 | | | m | 67 | rectum | pT2pN0M0 G3 | I |

**Table 2: PCR primer sequences and amplification settings**

| Accession Number | Gene | Forward Primer (t'-3') | Reverse Primer (5'-3') | Product Length (bp) | PCR Cycles | Annealing Temperature (C°) |
|---|---|---|---|---|---|---|
| NM_000545 | HNP1 | TCTACAACTGGTTTGCCAACC | GGCTTCTGTACTCAGCAGGC | 310 | 38 | 55 |
| NM_57733 | HNP1α | CCGCAGACTATGCTCATCAC | TCTGGGTGGAGATGAAGGTC | 331 | 39 | 55 |
| X58840.1 | HNF1β | CCTCTCCTCCAAACAAGCTG | GACTCCAGAGAGGGGTGTCA | 302 | 34 | 55 |
| AF147787 | Foxa2 IHNF381 | ATTGCTGGTCGTTTGTTGTG | TACGTGTTCATGCCGTTCAT | 187 | 39 | 55 |
| NN_022180 | HNF4 | GCCTGCCTCAAAGCCATCAT | GACCCTCCAAGCAGCATCTC | 370 | 34 | 55 |
| X87870 | HNF4α | CGTGGATCCTGGCAGATGATCGAGCAGATC | ACGGATCCTCTAGACAGGTTAAGCAACTT | 219 | 39 | 55 |
| NM_004133 | HNF4γ | GTCTTGGTGGAATGGGCTAA | AACCGATCTGCACTTGGAAC | 364 | 37 | 55 |
| AH007195 | NHF6 | GGGCAGATGGAAGAGATCAA | TGCGTTCATGAAGAAGTTGC | 449 | 37 | 55 |
| NM_004364 | C/EBPα | CCACGCCTGTCCTTAGAAAG | ATGGACTGATCGTGCTTCGT | 399 | 37 | 57 |
| XM_009178 | C/EBPγ | GATATCGCAGCAAAACAGCA | GTCGCCATCTGCTGTCGTAT | 430 | 37 | 55 |
| DQ246833 | MitATPase | CTAAAGGACGAACCTGA | TGGCCTGCAGTAATGTT | 315 | 30 | 55 |
| NM_005036 | PPARα | CTGGAAGCTTTGGCTTTACG | CGACAGAAAGGCACTTGTGA | 357 | 35 | 57 |
| X56774 | Igfβ | TGGATGCTCTTCAGTTCGTG | GATGTGTCTTTGGCCAACCT | 294 | 38 | 60 |
| NM_001621 | AHR | CTGCCTTTCCCACAAGATGT | GAAATTCAGCTCGGTCTTCG | 352 | 31 | 57 |
| NM_020999 | NGN3 m | CCCTCTACTCCCCAGTCTCC | CCTTACCCTTAGCACCCACA | 176 | 39 | 55 |
| NM_000691 | ALDH3A1 | TCAGCAGGACGAGCTCTACA | TCCACGTAGCAGGGACTCTT | 381 | 39 | 55 |
| NM_000667 | ADH1A1 | TTTCCATTGAGGAGGTGGAG | TTGCTCTCCGGGTTTTTACA | 265 | 36 | 55 |
| NM_000093 | CoI5A1 | TCTCCCGTCTTCCTCTACGA | AAACACGATGATGCCATTGA | 211 | 36 | 55 |
| NM_008786 | Cyp51 | TACCTTCTGGGGAGTGATGC | TTCCATGCAAACAATGGCTA | 319 | 36 | 55 |
| NM_000463 | UGT1A1 | ATGGCAATTGCTGATGCTTT | TCCAGCTCCCTTAGTCTCCA | 273 | 35 | 55 |
| NM_006644 | HSP105B | TGACCCCTTCATTCAAAAGG | CCAACAATCTGTGCAGCATC | 264 | 35 | 55 |
| M74099 | CDP | CACCTCAAAGCTGGAGGAAG | CGGCCAACTCAACTTCTAGG | 323 | 34 | 57 |
| NM_007052 | GATA4 | GTGTGTCAACTGTGGGGCTA | CCGTGGAGCTTCATGTAGAG | 292 | 36 | 55 |

**Table 3: Shift probe sequences**

| **Oligonucleotide Name** | **Sequence** |
|---|---|
| HNF6 | 5'-GATTCCATATTGATTTCAAAA-3' |
| NGN3 | 5'-GCTTGGTGCCAAATCCATGTGTCAGCTTCT-3' |
| Foxa2 (HNF3β) | 5'-GTTGACTAAGTCAATAATCAGAATCAG-3' |
| HNF4 | 5'-GGAAAGGGTCCAAAGGGGCGCCTTG-3' |
| TTR | 5'-GTTGACTAAGTCAATAATCAGAA-3' |
| PEPCK | 5'-CAAAGTTTAGTCAATCAAACGTT-3' |

**Table 4: PCR Primer sequences for HNF6 DNA sequence analysis**

| **Gene** | **Forward Primer (5'-3')** | **Reverse Primer (5'-3')** |
|---|---|---|
| HNF6-x1-1 | agaggaaggaaggcaacagtc | gtgaagctaccgctcacgttg |
| HNF6-x1-2 | atctccacagtctcggacaagt | gatctcttccatctgccctgaa |
| HNF6-x1-3 | gacaagatgctcacccccaac | tctcctacccttcctcctttg |
| HNF6-x2 | gcagaggtcagcaaacagaaagc | ctgctatcttgaggtcctggtct |

**Table 5: Gene expression of HNF6 target genes in colorectal liver metastases. Note, most genes were highly repressed.**

| **Function** | **name sience** | **Gene-titel** | **fold change** |
|---|---|---|---|
| ***metabolism*** | ADH1A | alcohol dehydrogenase 1A (class I) alpha polypeptide | -6,9 |
| | ADH1B | alcohol dehydrogenase 1B (class n beta polypeptide | -9,0 |
| | ALDH5A1 | aldehyd dehydrogenase 5 family member A1 | -3,6 |
| | AKR1C4 | aido-keto reductase family, member C4 | -11,1 |
| | BF | 8-factor, properdin | -7,8 |
| | BCKDHA | brached chain keto acid dehydrogenase E1, alpha polypeptide | -2,1 |
| | G6PC | glucose-6-phosphatase, catalytic | -9,4 |
| | HNMT | histamine N-methyltransferase | -2,3 |
| | IF | I factor (complement) | -8,1 |
| | ITIH1 | inter-alpha (globulin) inhibitor H1 | -9,2 |
| | PON1 | paraoxonase1 | -5,4 |
| | PCK1 | phosphoenolpyruvate carboxykionase 1 (soluble) | -8,7 |
| | UGT2B15 | UDP glycosyltransferase 2 familiy, polypeptide B15 | -5,8 |
| ***proteinsynthesis*** | AMPB | alpha-1-microglobulin/bikunin precursor | -8,5 |
| | APCS | amyloid P component serum | -7,8 |
| | APOH | apolipoprotein H (beta-2-glycoprotein I) | -7,4 |
| | F9 | coagulation factor IX (plasma thromboplastic component) | -12,3 |
| | F11 | coagulation factor IX (plasma thromboplastin antecedent) | -11,4 |
| | C1S | complement component 1, s subcomponent | -7,0 |
| | C2 | complement component 2 | -5,4 |
| | C8B | complement component 8, beta polypeptide | -10,6 |
| ***receptors transporters*** | AGTR1 | angiotensin II receptor, type 1 | -4,4 |
| | ABCA8 | ATP-binding cassette, subfamily A (ABC:) member 8 | -5,6 |
| | ABCB11 | ATP-binding cassette, subfamily B (MDR/TAP) member 11 | -4,7 |
| ***apoptosis*** | CRADD | CASP2 and RIPK domain containing adaptor with death domain | -3.4 |
| ***promotor of tumour growth*** | CXCL1 | chemokine ligand I (melanoma growth stimulating activity, alpha) | 5.0 |
| | CXCL3 | chemokine (C-X-C motif) ligand 3 | 4,0 |
| | FABP5 | fatty acid binding protein (psoriasis associated) | 4,1 |

The invention allows for the first time the rapid and effective identification of therapeutical targets in secondary tumors, in particular of transcription factors being influenced by the ambient tissue of the organ affected by the secondary tumor.
For example, HNF6 has been identified according to the inventive method.
The targets identified, in particular the genes and/or gene products of thereof, are used for screening and identifying drugs against secondary tumors by means of standard methods or by any other method known for identifying drugs regulating therapeutical targets, in particular regulating genes and/or gene products. For example, the PathwayStudio Database can be used for screening and identifying compounds, which inhibit proteins, in particular inhibit kinases regulating the identified targets, and which are tested by *in vitro, in situ* and/or *in vivo* studies, and which can be used for manufacturing a medicament against secondary tumors.
The drugs and/or compounds being identified or any other soluble substance having affinity with one of the therapeutical targets, in particular with a transcript and/or gene product of thereof, are produced and are linked with a marker, according to standard methods or any other known method, for identifying, labelling and treating of secondary metastases. For example, tagged antibodies are produced against human MHC class I molecules being in complex with a fragment of HNF6 and/or Foxa2 and are used for targeting secondary tumors.

### Legends to Figures

Figure 1:
   Haematoxylin and eosin staining of colorectal liver metastases of patient 30.
Figure 2:
   CT scan and intraoperative finding of colorectal liver metastases.
Figure 3:
   Expression of MitATPase (housekeeping gene) in healthy liver and colorectal liver metastases (ethidium bromide stained RT-PCR gel).
Figure 4:
   Gene expression in healthy liver and colorectal liver metastases - box blot.
Figure 5:
   Expression of liver enriched transcription factors and downstream target genes in healthy liver and colorectal liver metastases (ethidium bromide stained RTPCR gel).
Figure 6:
   Expression of the HNF6 target genes ADH1A1 and UGT1A1 for n=29 individual patients. X-axis: HNF6 gene expression, Y-axis (A) ADH1A1 expression, (B) UGT1A1 expression; Values are ratios, gene expression data relative to expression of the housekeeping gene mitochondrial ATPase.
Figure 7:
   Gene expression in healthy colon and primary colorectal cancer - box blot.
Figure 8:
   Expression of liver enriched transcription factors and downstream target genes in healthy colon and primary colorectal cancer (ethidium bromide stained RTPCR gel).
Figure 9:
   Western Blotting of HNF6 expression in human liver and colorectal liver Metastases.
Figure 10:
   Western Blotting of Foxa2 (A), HNF1β (B), C/EBPa (C) with nuclear extracts of healthy liver and colorectal liver metastases; Foxa2 in healthy colon and colonic cancer (D).
Figure 11:
   Electrophoretic mobilitiy shift assay:
      A: HNF6 DNA binding and competition assay with nuclear extracts of healthy Liver.
      B: HNF6 DNA binding in healthy liver, colorectal liver metastases, healthy colon and colonic cancer.
Figure 12:
   Electrophoretic mobilitiy shift assay:
      A: NGN3 DNA binding with nuclear extracts of healthy liver and colorectal liver metastases.
      B: Foxa2 DNA binding with nuclear extracts of healthy liver and colorectal liver metastases.
      C: Foxa2 DNA binding with nuclear extracts of healthy colon and colonic Cancer.
Figure 13:
   Electrophoretic mobilitiy shift assay: HNF6 DNA binding - search for isoform specificity.
Figure 14:
   Electropherogram showing sequences of genomic DNA extracts in healthy liver and colorectal liver metastases. Note we display the CBP binding and lysine acetylation site.
Figure 15:
   Hierarchical gene cluster analysis of liver enriched transcription factors and
   HNF6 target genes in healthy liver and colorectal liver metastases.

## Claims

1. Use of the non-acetylated form of HNF6 as marker for identifying or detecting liver metastases of colorectal cancer.

2. Method for identifying/detecting liver metastases from colorectal cancer in a liver sample, wherein the presence of the non-acetylated form of HNF6 is used as a positive marker for the presence of said metastases.

3. The method of claim 2, wherein the identification step is carried out by Western blot.

4. The method of claim 2 or 3, wherein the presence of the non-acetylated form of HNF6 is determined in a total protein extract isolated from the tissue of a liver sample.

## Patentansprüche

1. Verwendung der nicht-acetylierten Form von HNF6 als Marker zur Identifizierung oder Detektion von Lebermetastasen aus Dickdarmkrebs.

2. Verfahren zur Identifizierung/Detektion von Leber-Metastasen aus Dickdarmkrebs in einer Leberprobe, wobei das Vorhandensein der nicht-acetylierten Form von HNF6 als Positivmarker für das Vorliegen der besagten Metastasen verwendet wird.

3. Verfahren nach Anspruch 2, wobei der Identifizierungsschritt mittels Western Blot durchgeführt wird.

4. Verfahren nach Anspruch 2 oder 3, wobei das Vorhandensein der nicht-acetylierten Form von HNF6 in einem aus dem Gewebe einer Leberprobe isolierten Gesamtproteinextrakt bestimmt wird.

## Revendications

1. Utilisation de HNF6 de la forme non-acétylée comme marqueur pour identifier ou détecter des métastases au foie d'un cancer côlorectal.

2. Procédé pour identifier/détecter des métastases au foie d'un cancer côlorectal dans un échantillon de foie, en utilisant la présence de HNF6 sous forme non-acétylée comme marqueur positif pour la présence de lesdites métastases.

3. Procédé selon la revendication 2, dans lequelle on utilise le technique western blot pour identifier de lesdites métastases.

4. Procédé selon la revendication 2 ou 3, dans lequelle la présence de HNF6 sous forme non-acétylée est déterminée dans un extrait de protéine total qui était isolé d'un tissu d'échantillon de foie.
